# EUROPÄISCHE PATENTSCHRIFT

(11) **EP 3 223 067 B1**
(45) Veröffentlichungstag und Bekanntmachung des Hinweises auf die Patenterteilung: **26.02.2025**
(21) Anmeldenummer: 16161958.0
(22) Anmeldetag: 23.03.2016
(51) Int. Cl.: A61F 9/02, G02C 7/10

(54) **SONNENSCHUTZVORRICHTUNG**
SUNSHADE DEVICE
DISPOSITIF DE PROTECTION SOLAIRE

(43) Veröffentlichungstag der Anmeldung: 27.09.2017
(73) Patentinhaber: Optrel Holding AG, 9630 Wattwil (CH)
(72) Erfinder: DR. HOFER KRANER, Ramon, 9100 Herisau (CH); ESPOSITO, Martin, 8640 Rapperswil (CH)
(74) Vertreter: Daub, Thomas

(56) Entgegenhaltungen:
- WO-A1-2014/079574
- GB-A- 2 445 365
- US-A- 5 552 841
- US-A1- 2009 204 291
- US-B1- 7 970 172

## Beschreibung

### Stand der Technik

Die Erfindung betrifft eine Sonnenschutzvorrichtung, insbesondere eine Sonnenbrille.

Aus der US 8,081,262 B1 ist bereits eine Sonnenschutzvorrichtung mit zumindest einem optischen Sonnenschutzfilter, der zumindest eine Flüssigkristallzelle aufweist, mit zumindest einem Sensor, welcher zu einer Erfassung einer Sonneneinstrahlung vorgesehen ist, und mit zumindest einer Steuer- und/oder Regeleinheit, welche dazu vorgesehen ist, abhängig von einer Sonneneinstrahlung eine Durchlässigkeit des optischen Sonnenschutzfilter zu steuern und/oder zu regeln, vorgeschlagen worden.

Die Aufgabe der Erfindung besteht insbesondere darin, eine gattungsgemäße Vorrichtung mit verbesserten Eigenschaften hinsichtlich eines Komforts sowie hinsichtlich einer Nutzbarkeit bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer Sonnenschutzvorrichtung, insbesondere von einer Sonnenbrille, mit zumindest einem optischen Sonnenschutzfilter, der zumindest eine Flüssigkristallzelle aufweist, mit zumindest einer Sensoreinheit, welche zu einer Erfassung einer Sonneneinstrahlung vorgesehen ist, und mit zumindest einer Steuer- und/oder Regeleinheit, welche dazu vorgesehen ist, abhängig von einer Sonneneinstrahlung eine Durchlässigkeit des optischen Sonnenschutzfilter zu steuern und/oder zu regeln.

Es wird vorgeschlagen, dass die Steuer- und/oder Regeleinheit in zumindest einem Betriebszustand dazu vorgesehen ist, die zumindest eine Flüssigkristallzelle des optischen Sonnenschutzfilters zu einer Erzeugung eines für einen Benutzer definierten Durchlässigkeitsverlaufs mit zumindest zwei differierenden Durchlässigkeiten

anzusteuern. Die Steuer- und/oder Regeleinheit ist in zumindest einem Betriebszustand dazu vorgesehen, die zumindest eine Flüssigkristallzelle des optischen Sonnenschutzfilters zu einer Erzeugung eines aus einer Blickrichtung des Benutzers, insbesondere des Trägers, definierten Durchlässigkeitsverlaufs mit zumindest zwei differierenden Durchlässigkeiten anzusteuern. Die Steuer- und/oder Regeleinheit ist in zumindest einem Betriebszustand dazu vorgesehen, die zumindest eine Flüssigkristallzelle des optischen Sonnenschutzfilters zu einer Erzeugung eines blickwinkelabhängigen Durchlässigkeitsverlaufs anzusteuern. Die Flüssigkristallzelle wird in zumindest einem Betriebszustand zu einer Erzeugung einer blickwinkelabhängigen Durchlässigkeit angesteuert, welche aus einer Blickrichtung des Benutzers einen definierten Durchlässigkeitsverlauf ergibt. Erfindungsgemäß weist der zumindest eine optische Sonnenschutzfilter zumindest zwei Flüssigkristallzellen auf. Erfindungsgemäß ist jede Flüssigkristallzelle des optischen Sonnenschutzfilters jeweils einem Auge des Benutzers zugeordnet. Erfindungsgemäß wäre jedoch auch denkbar, dass der zumindest eine optische Sonnenschutzfilter lediglich eine Flüssigkristallzelle für beide Augen des Benutzers aufweist. Unter einer "Sonnenschutzvorrichtung" soll in diesem Zusammenhang insbesondere eine Vorrichtung verstanden werden, die zu einem Schutz der Augen eines Benutzers vor, insbesondere störender, Sonneneinstrahlung vorgesehen ist. Vorzugsweise soll darunter insbesondere eine Vorrichtung verstanden werden, welche dazu vorgesehen ist, eine Sonneneinstrahlung zumindest zu verringern. Besonders bevorzugt ist die Sonnenschutzvorrichtung in zumindest einem Betriebszustand zu einer Abdunkelung einer, insbesondere störender, Sonneneinstrahlung auf die Augen des Benutzers vorgesehen. Es sind verschiedene, einem Fachmann als sinnvoll erscheinende Ausbildungen der Sonnenschutzvorrichtung denkbar, wie beispielsweise als eine Sonnenschutzblende und/oder besonders bevorzugt als eine Sonnenbrille. Ferner soll in diesem Zusammenhang unter einem "optischen Sonnenschutzfilter" insbesondere ein optischer Filter verstanden werden, welcher insbesondere ein Schutzglas und/oder ein Kunststoffschutzglas, insbesondere zu einem Schutz vor störender Sonneneinstrahlung, bildet. Darunter soll insbesondere ein optischer Filter verstanden werden, dessen Lichtdurchlässigkeit einstellbar ausgeführt ist. Darunter soll insbesondere ein optischer Schutzfilter mit einer automatischen Verdunkelung verstanden werden. Der Sonnenschutzfilter weist zumindest eine in der Transmission schaltbare Flüssigkristallebene auf. Der optische Sonnenschutzfilter weist zumindest eine Flüssigkristallzelle auf. Es sind verschiedene, einem Fachmann als sinnvoll erscheinende Flüssigkristallzellen denkbar, wie insbesondere eine TN-Flüssigkristallzelle mit der Twisted-Nematic-Technik. Grundsätzlich wären jedoch auch eine andere, einem Fachmann als sinnvoll erscheinende Ausbildungen der Flüssigkristallzellen denkbar, wie beispielsweise als STN-Flüssigkristallzellen mit der Super-Twisted-Nematic-Technik, DSTN-Flüssigkristallzellen mit der Double-Super-Twisted-Nematic-Technik, TSTN-Flüssigkristallzellen mit der Triple-Super-Twisted-Nematic-Technik, VA-Flüssigkristallzellen mit der Vertical-Alignment-Technik, insbesondere PVA/MVA-Flüssigkristallzellen mit der Patterned-Vertical-Alignment- und/oder Multi-Domain-Vertical-Alignment-Technik, IPS-Flüssigkristallzellen mit der In-Plane-Switching-Technik, FLCD-Flüssigkristallzellen, also ferroelektrische Flüssigkristallzellen, und/oder TN-Flüssigkristallzellen mit der Guest-Host-Technik. Unter einer "Sensoreinheit" soll in diesem Zusammenhang insbesondere eine Einheit verstanden werden, die dazu vorgesehen ist, zumindest eine Kenngröße und/oder eine physikalische Eigenschaft aufzunehmen, wobei die Aufnahme aktiv, wie insbesondere durch Erzeugen und Aussenden eines elektrischen Messsignals, und/oder passiv, wie insbesondere durch eine Erfassung von Eigenschaftsänderungen eines Sensorbauteils, stattfinden kann. Es sind verschiedene, einem Fachmann als sinnvoll erscheinende Sensoreinheiten für eine Sonnenschutzvorrichtung denkbar. Vorzugsweise weist die Sensoreinheit zumindest eine Photozelle, insbesondere eine Photodiode und/oder insbesondere eine Solarzelle, auf. Bevorzugt ist die Photozelle insbesondere zumindest zu einer optischen Detektion eines Sonnenlichts und/oder eines künstlichen Lichts vorgesehen. Des Weiteren soll in diesem Zusammenhang unter einer "Steuer- und/oder Regeleinheit" insbesondere eine Einheit mit zumindest einer Steuerelektronik verstanden werden. Unter einer "Steuerelektronik" soll insbesondere eine Einheit mit zumindest einer elektronischen Schaltung verstanden werden, welche vorzugsweise aus Spannungs- und Vergleichsregelbausteinen besteht., Grundsätzlich kann die Steuerelektronik jedoch auch komplexer aufgebaut sein, wie insbesondere durch die Nutzung einer anwendungsspezifischen integrierten Schaltung (ASIC) und/oder eines Mikrokontrollerbausteins.

Unter einer "Durchlässigkeit des optischen Sonnenschutzfilters" soll in diesem Zusammenhang insbesondere ein Transmissionsgrad von Sonnenlicht durch den optischen Sonnenschutzfilter verstanden werden. Vorzugsweise soll darunter insbesondere verstanden werden, inwieweit das sichtbare Spektrum des Sonnenlichts durch den optischen Sonnenschutzfilter absorbiert und/oder reflektiert wird. Besonders bevorzugt soll darunter insbesondere eine Tönung verstanden werden. Ferner soll in diesem Zusammenhang unter einem "definierten Durchlässigkeitsverlauf" insbesondere ein für einen Benutzer, insbesondere einen Träger, sichtbarer örtlicher Verlauf einer Durchlässigkeit der Flüssigkristallzelle verstanden werden. Erfindungsgemäß soll darunter insbesondere ein aus einer Blickrichtung des Benutzers, insbesondere des Trägers, sichtbarer Verlauf einer Durchlässigkeit der Flüssigkristallzelle verstanden werden, welcher zu einem festen Zeitpunkt zumindest zwei differierende Durchlässigkeiten aufweist. Bevorzugt soll darunter insbesondere ein für einen Benutzer, insbesondere Träger, sichtbarer örtlicher Verlauf einer Durchlässigkeit der Flüssigkristallzelle verstanden werden, welcher durch die optischen Eigenschaften der Flüssigkristalle der Flüssigkristallzelle selbst erzeugt wird. Erfindungsgemäß entsteht der definierte Durchlässigkeitsverlauf dabei insbesondere durch die Winkelabhängigkeit der Flüssigkristallzelle, also eine Beeinflussung der Transmission der Flüssigkristallzelle abhängig von einem Blickwinkel des Auges des Benutzers auf die Kristallmoleküle sowie abhängig von der Orientierung der Kristallmoleküle. Mit zunehmender Abwinklung der Blickrichtung bzw. des Blickwinkels gegenüber den Kristallmolekülen der Flüssigkristallzelle sinkt insbesondere die Transmission der Flüssigkristallzelle und eine Absorption wird erhöht. Des Weiteren soll in diesem Zusammenhang unter "differierenden Durchlässigkeiten" insbesondere verstanden werden, dass die zumindest eine Flüssigkristallzelle zumindest aus Sicht des Benutzers, insbesondere des Trägers, zumindest zwei Bereiche aufweist, die aus einer Blickrichtung bzw. einem Blickwinkel des Benutzers, insbesondere des Trägers gegenüber einander eine wesentlich differierende Tönung aufweisen. Dabei soll unter einer "wesentlich differierenden Tönung" insbesondere eine Differenz zwischen den Tönungen von zumindest 1%, vorzugsweise von zumindest 10% und besonders bevorzugt von zumindest 30% verstanden werden. Die Tönung definiert dabei insbesondere eine prozentuale Lichtabsorption zumindest im Bereich von sichtbarem Licht, insbesondere in einem Wellenlängenbereich von 380 nm bis 780 nm. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Durch die erfindungsgemäße Sonnenschutzvorrichtung kann insbesondere eine für einen Benutzer vorteilhaft angenehme Abdunkelung erreicht werden. Insbesondere kann dem Benutzer durch den aus einer Blickrichtung des Benutzers auftretenden Durchlässigkeitsverlauf der Flüssigkristallzelle eine auf eine Sonneneinstrahlung angepasste Abdunkelung bereitgestellt werden. Es kann insbesondere ein hoher Komfort sowie eine vorteilhafte Nutzbarkeit der Sonnenschutzvorrichtung bereitgestellt werden.

Erfindungsgemäß ist die Steuer- und/oder Regeleinheit in zumindest einem Betriebszustand dazu vorgesehen, die zumindest eine Flüssigkristallzelle des optischen Sonnenschutzfilters zu einem Halten des für den Benutzer definierten Durchlässigkeitsverlaufs anzusteuern. Vorzugsweise ist die Steuer- und/oder Regeleinheit dazu vorgesehen, bei gleichbleibender Sonneneinstrahlung relativ zu der Sonnenschutzvorrichtung die zumindest eine Flüssigkristallzelle des optischen Sonnenschutzfilters zu einem Halten des aus einer Blickrichtung des Benutzers definierten Durchlässigkeitsverlaufs anzusteuern. Erfindungsgemäß soll unter einem "Halten des definierten Durchlässigkeitsverlaufs" in diesem Zusammenhang insbesondere ein Beibehalten des zu einem Zeitpunkt vorliegenden aus einer Blickrichtung des Benutzers definierten Durchlässigkeitsverlaufs über einen unbegrenzten Zeitraum hinweg verstanden werden. Vorzugsweise soll darunter insbesondere ein Beibehalten des zu einem Zeitpunkt vorliegenden aus einer Blickrichtung des Benutzers definierten Durchlässigkeitsverlaufs über einen Zeitraum hinweg, bei welchem eine Sonneneinstrahlung relativ zu der Sonnenschutzvorrichtung zumindest im Wesentlichen gleich bleibt, verstanden werden. Besonders bevorzugt soll darunter insbesondere ein Beibehalten der zu einem festen Zeitpunkt vorliegenden Ausrichtung der Kristallmoleküle der Flüssigkristallzelle, bei welcher aus einer Blickrichtung des Benutzers der definierte Durchlässigkeitsverlauf erzeugt wird, über einen Zeitraum hinweg, bei welchem eine Sonneneinstrahlung relativ zu der Sonnenschutzvorrichtung zumindest im Wesentlichen gleich bleibt, verstanden werden. Dadurch kann zuverlässig dauerhaft ein Durchlässigkeitsverlauf in der Flüssigkristallzelle beibehalten werden. Insbesondere kann dadurch dauerhaft ein aus der Blickrichtung des Benutzers sichtbarer Verlauf erzeugt werden. Hierdurch kann erreicht werden, dass einem Benutzer bei optimaler Abdunkelung eine maximale Sicht bereitgestellt wird.

Des Weiteren wird vorgeschlagen, dass für einen Benutzer eine geringere Durchlässigkeit der zumindest einen Flüssigkristallzelle bei dem Durchlässigkeitsverlauf oberhalb einer höheren Durchlässigkeit angeordnet ist. Erfindungsgemäß erstreckt sich der Durchlässigkeitsverlauf aus einer Blickrichtung des Benutzers von oben nach unten, wobei eine Durchlässigkeit von oben nach unten zunimmt. Eine Abdunkelung nimmt bei dem Durchlässigkeitsverlauf insbesondere aus einer Blickrichtung des Benutzers von oben nach unten ab. Dabei soll unter "oberhalb" insbesondere oberhalb in einer regulären Lage der Sonnenschutzvorrichtung verstanden werden. Vorzugsweise soll darunter insbesondere oberhalb in einem getragenen Zustand der Sonnenschutzvorrichtung bei einer aufrechten Haltung des Benutzers verstanden werden. Insbesondere soll darunter daher insbesondere oberhalb aus Sicht des Benutzers verstanden werden. Dadurch kann insbesondere eine für einen Benutzer vorteilhaft angenehme Abdunkelung erreicht werden. Vorzugsweise kann dadurch insbesondere bei einer aufrechten Haltung des Bedieners eine der Sonne zugewandte Oberseite der Flüssigkristallzelle, auf welche die Sonnenstrahlen auf die zumindest eine Flüssigkristallzelle einstrahlen, stärker abgedunkelt werden. Hierdurch kann erreicht werden, dass einem Benutzer bei optimaler Abdunkelung eine maximale Sicht bereitgestellt wird.

Erfindungsgemäß ist die Steuer- und/oder Regeleinheit in zumindest einem Betriebszustand dazu vorgesehen, eine Stärke und/oder Erstreckung des Durchlässigkeitsverlaufs der zumindest einen Flüssigkristallzelle des optischen Sonnenschutzfilter für einen Benutzer abhängig von einer Sonneneinstrahlung einzustellen. Vorzugsweise ist die Steuer- und/oder Regeleinheit in zumindest einem Betriebszustand dazu vorgesehen, eine Erstreckung des Durchlässigkeitsverlaufs der zumindest einen Flüssigkristallzelle des optischen Sonnenschutzfilters aus einer Blickrichtung des Benutzers abhängig von einer Sonneneinstrahlung einzustellen, wobei damit für einen Benutzer eine durchschnittliche Durchlässigkeit über die Fläche der zumindest einen Flüssigkristallzelle verändert wird. Bevorzugt ist die Steuer- und/oder Regeleinheit dazu vorgesehen, einen aus einer Blickrichtung des Benutzers definierten, festgelegten Durchlässigkeitsverlauf der zumindest einen Flüssigkristallzelle des optischen Sonnenschutzfilters einzustellen, wobei abhängig von einer Sonneneinstrahlung eine Position des Durchlässigkeitsverlaufs verändert wird. Mit zunehmender Sonneneinstrahlung verringert sich aus einer Blickrichtung des Benutzers insbesondere eine durchschnittliche Durchlässigkeit über die Fläche der zumindest einen Flüssigkristallzelle. Vorzugsweise erstreckt sich der Durchlässigkeitsverlauf aus einer Blickrichtung des Benutzers mit zunehmender Sonneneinstrahlung immer weiter, insbesondere von oben nach unten, in die Flüssigkristallzelle. Dadurch kann insbesondere eine für einen Benutzer vorteilhaft angenehme und angepasste Abdunkelung erreicht werden. Ferner kann erreicht werden, dass einem Benutzer bei optimaler Abdunkelung eine maximale Sicht bereitgestellt wird.

Alternativ oder zusätzlich wäre auch denkbar, dass die Steuer- und/oder Regeleinheit in zumindest einem Betriebszustand dazu vorgesehen ist, eine Stärke und/oder Erstreckung des Durchlässigkeitsverlaufs der zumindest einen Flüssigkristallzelle des optischen Sonnenschutzfilters für einen Benutzer abhängig von zumindest einer Benutzereingabe einzustellen. Vorzugsweise ist die Steuer- und/oder Regeleinheit in zumindest einem Betriebszustand dazu vorgesehen ist, eine Stärke und/oder Erstreckung des Durchlässigkeitsverlaufs der zumindest einen Flüssigkristallzelle des optischen Sonnenschutzfilters für einen Benutzer abhängig von einer benutzereinstellbaren Stufe zur Anpassung des Durchlässigkeitsverlaufs einzustellen. Bevorzugt erfolgt eine Benutzereingabe und/oder eine Eingabe der benutzereinstellbaren Stufe über eine Eingabeeinheit. Besonders bevorzugt definiert die Benutzereingabe dabei insbesondere einen Korrekturfaktor, einen Multiplikationsfaktor und/oder einen Extremwert, insbesondere einen Minimal- und/oder besonders bevorzugt einen Maximalwert, des für einen Benutzer definierten Durchlässigkeitsverlaufs, insbesondere relativ zu der Sonneneinstrahlung. Grundsätzlich wäre jedoch auch denkbar, dass der für einen Benutzer definierte Durchlässigkeitsverlauf mittels der Benutzereingabe von einem Benutzer auf einen definierten Wert festgelegt werden kann. Unter einer "Eingabeeinheit" soll in diesem Zusammenhang insbesondere eine Einheit verstanden werden, über welche, insbesondere von einem Bediener, Einstellungen getätigt werden können. Vorzugsweise soll darunter insbesondere eine Einheit verstanden werden, die dazu vorgesehen ist, bei einem Einstellvorgang eine Eingabegröße von einem Bediener aufzunehmen. Vorzugsweise ist die Einheit dazu vorgesehen, insbesondere unmittelbar von einem Bediener kontaktiert zu werden, wobei eine Berührung der Eingabeeinheit und/oder eine auf ein Bedienelement der Eingabeeinheit ausgeübte Betätigungskraft und/oder eine von einem Bediener geänderte Stellung der Eingabeeinheit sensiert wird. Grundsätzlich kann jedoch auch lediglich eine aktuelle Stellung der Eingabeeinheit erfasst werden. Dadurch kann der für einen Benutzer definierte Durchlässigkeitsverlauf insbesondere von einem Benutzer individuell angepasst und/oder eingestellt werden.

Es wird weiter vorgeschlagen, dass die Steuer- und/oder Regeleinheit in zumindest einem Betriebszustand dazu vorgesehen ist, eine Ansteuerspannung zu einer Ansteuerung der zumindest einen Flüssigkristallzelle des optischen Sonnenschutzfilter auf einen Wert unterhalb einer Sättigungsfeldstärke der zumindest einen Flüssigkristallzelle zu steuern und/oder zu regeln. Erfindungsgemäß ist die Steuer- und/oder Regeleinheit in zumindest einem Betriebszustand dazu vorgesehen, eine Ansteuerspannung zu einer Ansteuerung der zumindest einen Flüssigkristallzelle des optischen Sonnenschutzfilter auf einen Wert unterhalb einer zu einer Bereitstellung einer Sättigungsfeldstärke der zumindest einen Flüssigkristallzelle benötigten Sättigungsspannung zu steuern und/oder zu regeln. Erfindungsgemäß ist die Steuer- und/oder Regeleinheit dazu vorgesehen, eine Ansteuerspannung zu einer Ansteuerung der zumindest einen Flüssigkristallzelle des optischen Sonnenschutzfilters auf einen Wert unterhalb der Sättigungsspannung zu steuern und/oder zu regeln. Besonders bevorzugt nimmt mit zunehmender Ansteuerspannung der Unterschied zwischen einem hellen Bereich und dunklen Bereich der Flüssigkristallzelle zu. Solange die Ansteuerspannung unterhalb der zu einer Bereitstellung einer Sättigungsfeldstärke der zumindest einen Flüssigkristallzelle benötigten Sättigungsspannung betrieben wird, bleibt der Unterschied zwischen dem hellen und dunklen Bereich der Flüssigkristallzelle insbesondere bestehen. Sobald die Ansteuerspannung die zu einer Bereitstellung einer Sättigungsfeldstärke der zumindest einen Flüssigkristallzelle benötigte Sättigungsspannung erreicht, würde der Benutzer in einem oberen Bereich insbesondere wieder eine Aufhellung wahrnehmen. Unter einer "Ansteuerspannung" soll in diesem Zusammenhang insbesondere eine Spannung verstanden werden, mit welcher Elektroden, insbesondere Elektrodenschichten, der zumindest einen Flüssigkristallzelle zu einer Erzeugung eines elektrischen Felds zu einer Ausrichtung einer Flüssigkristallschicht der zumindest einen Flüssigkristallzelle beaufschlagt werden. Ferner soll in diesem Zusammenhang unter einer "Sättigungsfeldstärke" insbesondere eine minimale Grenzfeldstärke verstanden werden, welche zu einer zumindest annähernd vollständigen Ausrichtung, insbesondere Ordnung, der Flüssigkristallschicht der zumindest einen Flüssigkristallzelle benötigt wird. Vorzugsweise soll darunter insbesondere eine minimale Grenzfeldstärke verstanden werden, welche zu einer Induktion eines für eine vollständige Ausrichtung der Flüssigkristallschicht der zumindest einen Flüssigkristallzelle benötigten Dipolmoments benötigt wird. Dadurch kann vorteilhaft konstruktiv einfach ein Durchlässigkeitsverlauf der zumindest einen Flüssigkristallzelle des optischen Sonnenschutzfilters bereitgestellt werden. Ferner kann dadurch insbesondere bei optimaler Abdunkelung ein vorteilhaft geringer Energieverbrauch der Sonnenschutzvorrichtung erreicht werden.

Ferner wird vorgeschlagen, dass die zumindest eine Flüssigkristallzelle des optischen Sonnenschutzfilters zumindest teilweise gebogen ausgebildet ist. Vorzugsweise ist die zumindest eine Flüssigkristallzelle des optischen Sonnenschutzfilters über eine wesentliche Erstreckung gebogen ausgebildet. Bevorzugt ist die zumindest eine Flüssigkristallzelle des optischen Sonnenschutzfilters wesentlich gebogen ausgebildet. Unter "zumindest teilweise gebogen" soll in diesem Zusammenhang insbesondere verstanden werden, dass die Flüssigkristallzelle in zumindest einer Ebene betrachtet eine Krümmung aufweist, die von Null verschieden ist. Dabei soll unter einer "Krümmung" in einem Punkt einer Fläche, die von Null verschieden ist, in diesem Zusammenhang insbesondere eine quadratisch mit einem Abstand zu dem Punkt der Fläche anwachsende Abweichung zu einer Tangentialebene verstanden werden. Unter "wesentlich gebogen" soll in diesem Zusammenhang insbesondere verstanden werden, dass eine maximale Entfernung der Flüssigkristallzelle zu einer Tangentialebene in einem Mittelpunkt der Flüssigkristallzelle, senkrecht zu der Tangentialebene betrachtet, zumindest 2 mm, vorzugsweise zumindest 4 mm und besonders bevorzugt zumindest 6 mm beträgt. Dadurch kann insbesondere eine vorteilhaft hohe optische Qualität der Sonnenschutzvorrichtung bereitgestellt werden. Es kann insbesondere ein vorteilhaft geringer Winkelunterschied zwischen den Strahlengängen der einzelnen Augen erreicht werden. Es kann insbesondere eine irritierende Durchsicht vor allem in einem Randbereich der Sonnenschutzvorrichtung vermieden werden. Ferner kann eine hohe Ergonomie der Sonnenschutzvorrichtung erreicht werden. Insbesondere kann vermieden werden, dass Wimpern des Benutzers die zumindest eine Flüssigkristallzelle berühren.

Des Weiteren wird vorgeschlagen, dass die zumindest eine Flüssigkristallzelle des optischen Sonnenschutzfilters von einer Kunststoff-Flüssigkristallzelle gebildet ist. Vorzugsweise besteht die Flüssigkristallzelle zumindest teilweise aus einem Kunststoff, insbesondere aus Polykarbonat. Besonders bevorzugt besteht zumindest eine Begrenzungsscheibe der Flüssigkristallzelle aus einem Kunststoff, insbesondere aus Polykarbonat. Grundsätzlich wäre jedoch auch denkbar, dass die zumindest eine Flüssigkristallzelle des optischen Sonnenschutzfilters von einer Glas-Flüssigkristallzelle gebildet ist. Dabei wäre insbesondere denkbar, dass die zumindest eine Flüssigkristallzelle zumindest teilweise aus gebogenem Glas gefertigt ist. Dadurch kann vorteilhaft ein Gewicht der Sonnenschutzvorrichtung gering gehalten werden. Ferner kann durch die Verwendung von Kunststoff-Flüssigkristallzellen ein vorteilhaft hoher UV-Schutz bereitgestellt werden, insbesondere durch die Absorption im Kunststoff, insbesondere im Polykarbonat.

Ferner wird vorgeschlagen, dass die Sonnenschutzvorrichtung einen Brillenrahmen aufweist, welcher zu einer Aufnahme des optischen Sonnenschutzfilters vorgesehen ist. Vorzugsweise weist der Brillenrahmen zwei Aufnahmebereiche auf, die jeweils zu einer Aufnahme einer Flüssigkristallzelle des zumindest einen optischen Sonnenschutzfilters vorgesehen ist. Unter einem "Brillenrahmen" soll in diesem Zusammenhang insbesondere ein Rahmen verstanden werden, welcher zu einer Anordnung der Sonnenschutzvorrichtung in einem Gesicht des Benutzers vorgesehen ist. Vorzugsweise ist der Rahmen zu einer Anordnung der Sonnenschutzvorrichtung auf einer Nase sowie den Ohren des Benutzers vorgesehen. Besonders bevorzugt ist der Brillenrahmen von einem Brillengestell gebildet. Dadurch kann insbesondere eine vorteilhafte Ausbildung der Sonnenschutzvorrichtung erreicht werden. Vorzugsweise kann die Sonnenschutzvorrichtung dadurch zuverlässig in einem Gesicht des Benutzers angeordnet werden. Es kann insbesondere eine brillenartige Ausbildung der Sonnenschutzvorrichtung bereitgestellt werden.

Erfindungsgemäß ist die zumindest eine Sensoreinheit in zumindest einem Betriebszustand zu einer vollständigen Bereitstellung einer Energie zu einer Ansteuerung der zumindest einen Flüssigkristallzelle des optischen Sonnenschutzfilters vorgesehen. Während eines Betriebs der Sonnenschutzvorrichtung ist die zumindest eine Sensoreinheit zu einer vollständigen Bereitstellung einer Energie zu einer Ansteuerung der zumindest einen Flüssigkristallzelle des optischen Sonnenschutzfilters vorgesehen.

Während eines Betriebs der Sonnenschutzvorrichtung ist die zumindest eine Sensoreinheit zu einer vollständigen Gewinnung einer zu einer Ansteuerung der zumindest einen Flüssigkristallzelle des optischen Sonnenschutzfilters benötigten Energie vorgesehen. Erfindungsgemäß ist die Sonnenschutzvorrichtung batterielos ausgebildet. Dadurch kann insbesondere auf eine externe Energieversorgung wie beispielsweise mittels einer Batterie und/oder mittels eines Akkumulators verzichtet werden. Es kann eine autarke Sonnenschutzvorrichtung bereitgestellt werden.

Es wird weiter vorgeschlagen, dass die zumindest eine Sensoreinheit zumindest eine Photodiode aufweist. Vorzugsweise weist die Sensoreinheit zumindest eine Solarzelle auf. Bevorzugt ist die Sensoreinheit von einer Solarzelle und/oder Photozelle gebildet. Dadurch kann eine vorteilhaft zuverlässige Sensoreinheit mit einer vorteilhaft hohen Energieausbeute bereitgestellt werden. Ferner kann insbesondere auf eine externe Energieversorgung wie beispielsweise mittels einer Batterie und/oder mittels eines Akkumulators verzichtet werden. Bei der Verwendung einer Photozelle kann insbesondere eine Sensoreinheit bereitgestellt werden, mittels der insbesondere auch Licht in einem nicht sichtbaren Bereich, wie insbesondere Infrarotlicht, erfasst und vorzugsweise verwertet werden kann. Es kann eine autarke Sonnenschutzvorrichtung bereitgestellt werden. Insbesondere kann dadurch erreicht werden, dass mit zunehmender Sonneneinstrahlung mehr Energie zu einer Ansteuerung der zumindest einen Flüssigkristallzelle des optischen Sonnenschutzfilters zur Verfügung steht. Eine Energieerzeugung ist dadurch vorteilhaft an einen Energiebedarf angepasst.

Ferner geht die Erfindung aus von einem Verfahren zu einem Betrieb der Sonnenschutzvorrichtung. Es wird vorgeschlagen, dass die zumindest eine Flüssigkristallzelle des optischen Sonnenschutzfilters zu einer Erzeugung eines für einen Benutzer definierten Durchlässigkeitsverlaufs angesteuert und/oder geregelt wird. Erfindungsgemäß wird die zumindest eine Flüssigkristallzelle des optischen Sonnenschutzfilters zu einer Erzeugung eines aus einer Blickrichtung des Benutzers definierten Durchlässigkeitsverlaufs mit zumindest zwei differierenden Durchlässigkeiten angesteuert. Dadurch kann insbesondere eine für einen Benutzer vorteilhaft angenehme Abdunkelung erreicht werden. Insbesondere kann dem Benutzer durch den aus einer Blickrichtung des Benutzers auftretenden Durchlässigkeitsverlauf der Flüssigkristallzelle eine auf eine Sonneneinstrahlung angepasste Abdunkelung bereitgestellt werden. Hierdurch kann erreicht werden, dass einem Benutzer bei optimaler Abdunkelung eine maximale Sicht bereitgestellt wird. Es kann insbesondere ein hoher Komfort sowie eine vorteilhafte Nutzbarkeit der Sonnenschutzvorrichtung bereitgestellt werden.

Des Weiteren wird vorgeschlagen, dass die zumindest eine Flüssigkristallzelle des optischen Sonnenschutzfilters zu einer Erzeugung eines für einen Benutzer definierten Durchlässigkeitsverlaufs abhängig von einer Sonneneinstrahlung angesteuert und/oder geregelt wird. Erfindungsgemäß wird die zumindest eine Flüssigkristallzelle des optischen Sonnenschutzfilters zu einer Erzeugung eines aus einer Blickrichtung des Benutzers definierten Durchlässigkeitsverlaufs abhängig von einer Sonneneinstrahlung angesteuert. Dadurch kann insbesondere eine für einen Benutzer vorteilhaft angenehme Abdunkelung erreicht werden. Insbesondere kann dadurch ein Durchlässigkeitsverlauf an eine aktuelle Sonneneinstrahlung angepasst werden. Es kann insbesondere eine individuelle Anpassung des Durchlässigkeitsverlaufs an eine Sonneneinstrahlung erreicht werden.

Es wird ferner vorgeschlagen, dass die zumindest eine Flüssigkristallzelle des optischen Sonnenschutzfilters zu einer Erzeugung eines für einen Benutzer definierten Durchlässigkeitsverlaufs abhängig von zumindest einer Benutzereingabe angesteuert und/oder geregelt wird. Vorzugsweise erfolgt eine Benutzereingabe über eine Eingabeeinheit. Besonders bevorzugt definiert die Benutzereingabe dabei insbesondere einen Korrekturfaktor, einen Multiplikationsfaktor und/oder einen Extremwert, insbesondere einen Minimal- und/oder besonders bevorzugt einen Maximalwert, des für einen Benutzer definierten Durchlässigkeitsverlaufs, insbesondere relativ zu der Sonneneinstrahlung. Grundsätzlich wäre jedoch auch denkbar, dass der für einen Benutzer definierte Durchlässigkeitsverlauf mittels der Benutzereingabe von einem Benutzer auf einen definierten Wert festgelegt werden kann. Dadurch kann der für einen Benutzer definierte Durchlässigkeitsverlauf insbesondere von einem Benutzer individuell angepasst und/oder eingestellt werden. Hierdurch kann erreicht werden, dass einem Benutzer bei optimaler Abdunkelung eine maximale Sicht bereitgestellt wird. Es kann insbesondere ein hoher Komfort sowie eine vorteilhafte Nutzbarkeit der Sonnenschutzvorrichtung bereitgestellt werden.

Es wird weiter vorgeschlagen, dass die zumindest eine Flüssigkristallzelle des optischen Sonnenschutzfilters zu einer Erzeugung eines für einen Benutzer definierten Durchlässigkeitsverlaufs abhängig von einer benutzereinstellbaren Stufe zur Anpassung des Durchlässigkeitsverlaufs angesteuert und/oder geregelt wird. Vorzugsweise ist die Stufe über eine Eingabeeinheit auswählbar. Besonders bevorzugt definiert die benutzereinstellbare Stufe dabei insbesondere einen Korrekturfaktor und/oder einen Multiplikationsfaktor des für einen Benutzer definierten Durchlässigkeitsverlaufs, insbesondere relativ zu der Sonneneinstrahlung. Dadurch kann der für einen Benutzer definierte Durchlässigkeitsverlauf insbesondere von einem Benutzer individuell angepasst und/oder eingestellt werden. Hierdurch kann erreicht werden, dass einem Benutzer bei optimaler Abdunkelung eine maximale Sicht bereitgestellt wird. Es kann insbesondere ein hoher Komfort sowie eine vorteilhafte Nutzbarkeit der Sonnenschutzvorrichtung bereitgestellt werden.

Die erfindungsgemäße Sonnenschutzvorrichtung sowie das Verfahren sollen hierbei nicht auf die oben beschriebene Anwendung und Ausführungsform beschränkt sein. Insbesondere können die erfindungsgemäße Sonnenschutzvorrichtung sowie das Verfahren zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine erfindungsgemäße Sonnenschutzvorrichtung mit einem Sonnenschutzfilter, welcher zwei Flüssigkristallzellen aufweist, mit einer Sensoreinheit und mit einer Steuer- und Regeleinheit und einen Benutzer, welcher die erfindungsgemäße Sonnenschutzvorrichtung trägt in einer schematischen Frontalansicht,
- Fig. 2: die erfindungsgemäße Sonnenschutzvorrichtung und den Benutzer, welcher die erfindungsgemäße Sonnenschutzvorrichtung trägt in einer schematischen Seitenansicht,

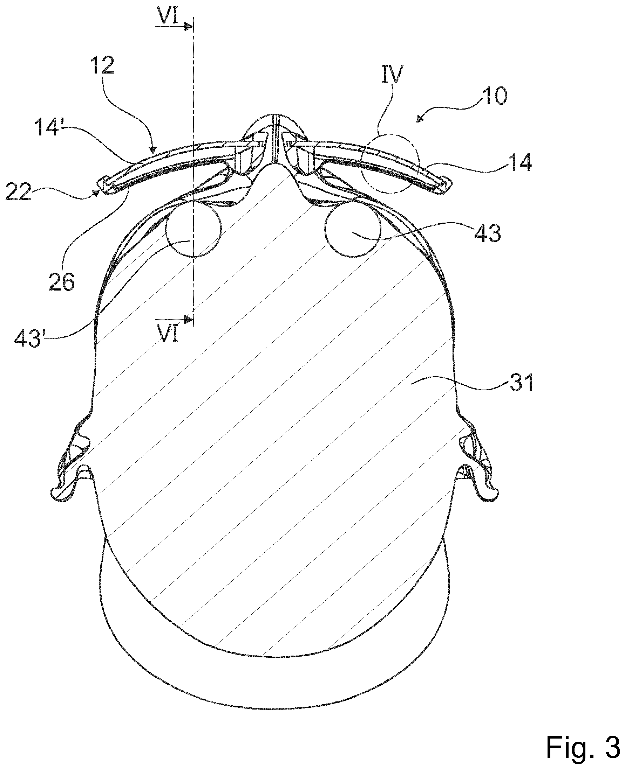

- Fig. 3: die erfindungsgemäße Sonnenschutzvorrichtung und den Benutzer, welcher die erfindungsgemäße Sonnenschutzvorrichtung trägt, in einer schematischen Schnittdarstellung entlang der der Schnittlinie III,
- Fig. 4: einen Teilausschnitt IV-IV der erfindungsgemäßen Sonnenschutzvorrichtung mit dem Sonnenschutzfilter in einer schematischen Schnittdarstellung,
- Fig. 5: die erfindungsgemäße Sonnenschutzvorrichtung und den Benutzer in einer schematischen Schnittdarstellung entlang der Schnittlinie III mit verschiedenen Blickwinkeln des Benutzers,
- Fig. 6: die erfindungsgemäße Sonnenschutzvorrichtung und den Benutzer in einer schematischen Schnittdarstellung entlang der Schnittlinie VI mit verschiedenen Blickwinkeln des Benutzers,
- Fig. 7: ein Diagramm einer Transmission der Flüssigkristallzellen des Sonnenschutzfilters abhängig von einer Ansteuerspannung,
- Fig. 8A: einen Durchlässigkeitsverlauf der Flüssigkristallzellen der erfindungsgemäßen Sonnenschutzvorrichtung in einem ersten Zustand,
- Fig. 8B: einen Durchlässigkeitsverlauf der Flüssigkristallzellen der erfindungsgemäßen Sonnenschutzvorrichtung in einem zweiten Zustand,
- Fig. 8C: einen Durchlässigkeitsverlauf der Flüssigkristallzellen der erfindungsgemäßen Sonnenschutzvorrichtung in einem dritten Zustand,
- Fig. 8D: einen Durchlässigkeitsverlauf der Flüssigkristallzellen der erfindungsgemäßen Sonnenschutzvorrichtung in einem vierten Zustand,
- Fig. 9: ein Ablaufdiagramm eines Verfahrens zu einem Betrieb der erfindungsgemäßen Sonnenschutzvorrichtung und
- Fig. 10: einen schematischen Schaltplan der Steuer- und Regeleinheit der erfindungsgemäßen Sonnenschutzvorrichtung.

### Beschreibung des Ausführungsbeispiels

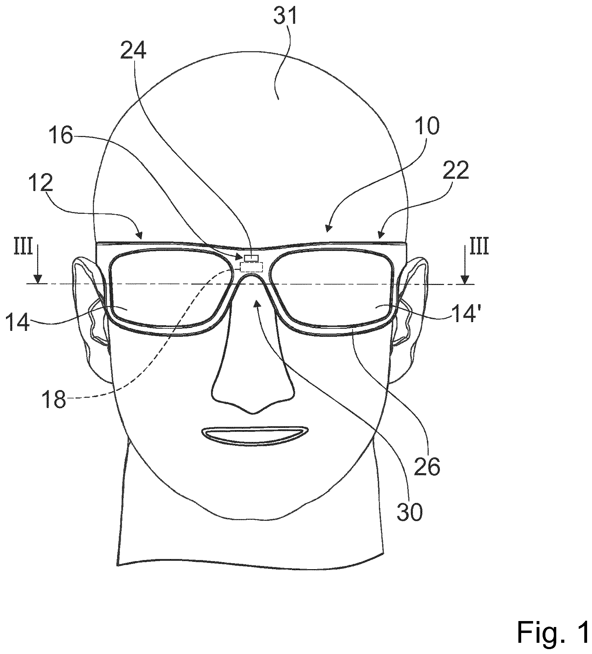

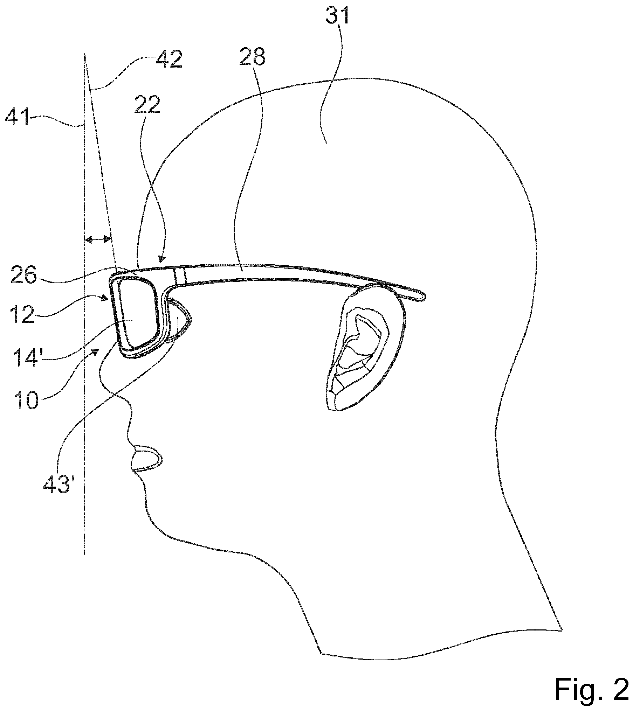

Die Figuren 1 und 2 zeigen eine Sonnenschutzvorrichtung 10. Die Sonnenschutzvorrichtung 10 ist von einer Sonnenbrille gebildet. Die Sonnenschutzvorrichtung 10 ist von einer Sonnenbrille gebildet, welche selbsttätig abhängig von einer Sonneneinstrahlung abdunkelt. Grundsätzlich wäre jedoch auch eine andere, einem Fachmann als sinnvoll erscheinende Ausbildung der Sonnenschutzvorrichtung 10 denkbar. Die Sonnenschutzvorrichtung 10 weist einen Brillenrahmen 22 auf. Der Brillenrahmen 22 ist von einem Brillengestell gebildet. Der Brillenrahmen 22 besteht im Wesentlichen aus Kunststoff. Grundsätzlich wäre jedoch auch ein anderes, einem Fachmann als sinnvoll erscheinendes Material denkbar. Der Brillenrahmen 22 weist einen Grundrahmen 26 sowie zwei an dem Grundrahmen 26 beweglich gelagerte Brillenbügel 28 auf, von denen lediglich einer sichtbar ist. Der Grundrahmen 26 weist einen Nasenausschnitt 30 zu einer Auflage auf einer Nase eines Benutzers 31 auf. Die Brillenbügel 28 sind jeweils zu einer Auflage auf den Ohren des Benutzers 31 vorgesehen.

Ferner weist die Sonnenschutzvorrichtung 10 einen optischen Sonnenschutzfilter 12 auf. Der Brillenrahmen 22 ist zu einer Aufnahme des optischen Sonnenschutzfilters 12 vorgesehen. Der optische Sonnenschutzfilter 12 ist in dem Brillenrahmen 22 aufgenommen. Eine Lichtdurchlässigkeit des optischen Sonnenschutzfilters 12 ist einstellbar ausgeführt. Der optische Sonnenschutzfilter 12 ist im Wesentlichen Transparent ausgebildet, wobei eine Transmission T des optischen Sonnenschutzfilters 12 elektrisch veränderbar ausgebildet ist. Der optische Sonnenschutzfilter 12 weist zwei Flüssigkristallzellen 14, 14' auf. Die Flüssigkristallzellen 14, 14' sind gegenübereinander spiegelsymmetrisch ausgebildet. Die Flüssigkristallzellen 14, 14' sind in dem Brillenrahmen 22 aufgenommen. Der Grundrahmen 26 des Brillenrahmens 22 weist zwei Ausnehmungen auf, in welchen die Flüssigkristallzellen 14, 14' aufgenommen sind. Die Flüssigkristallzellen 14, 14' sind jeweils auf gegenüberliegenden Seiten des Nasenausschnitts 30 angeordnet. Jede der Flüssigkristallzellen 14, 14' des optischen Sonnenschutzfilters 12 ist jeweils einem Auge des Benutzers 31 zugeordnet. Die Flüssigkristallzellen 14, 14' weisen jeweils eine in der Transmission T schaltbare Flüssigkristallebene 32 auf.

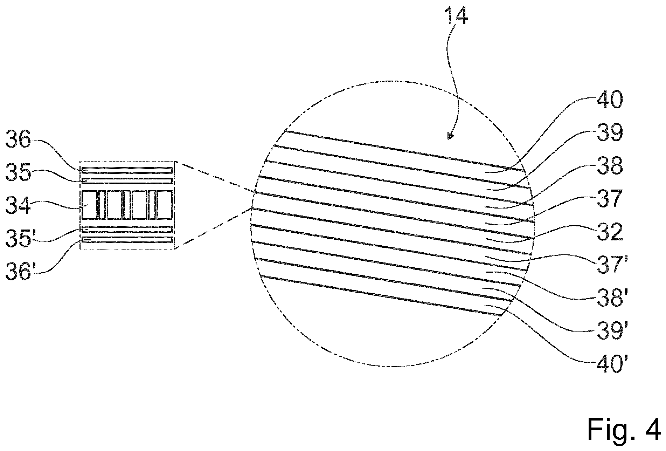

Die Flüssigkristallzellen 14, 14' des optischen Sonnenschutzfilters 12 sind jeweils von einer Kunststoff-Flüssigkristallzelle gebildet. Die Flüssigkristallzellen 14, 14' bestehen jeweils aus mehreren Schichten. Eine Anzahl von Schichten ist hierbei lediglich beispielhaft und kann grundsätzlich variieren. Die Flüssigkristallzellen 14, 14' sind jeweils von einer TN-Flüssigkristallzelle gebildet. Die Flüssigkristallzellen 14, 14' basieren daher auf der Twisted-Nematic-Technik. Grundsätzlich wären jedoch auch andere, einem Fachmann als sinnvoll erscheinende Ausbildung der Flüssigkristallzellen 14, 14' denkbar, wie beispielsweise als STN-Flüssigkristallzellen mit der Super-Twisted-Nematic-Technik, DSTN-Flüssigkristallzellen mit der Double-Super-Twisted-Nematic-Technik, TSTN-Flüssigkristallzellen mit der Triple-Super-Twisted-Nematic-Technik, VA-Flüssigkristallzellen mit der Vertical-Alignment-Technik, insbesondere PVA/MVA-Flüssigkristallzellen mit der Patterned-Vertical-Alignment- und/oder Multi-Domain-Vertical-Alignment-Technik, IPS-Flüssigkristallzellen mit der In-Plane-Switching-Technik, FLCD-Flüssigkristallzellen, also ferroelektrische Flüssigkristallzellen, und/oder TN-Flüssigkristallzellen mit der Guest-Host-Technik. Die Flüssigkristallzellen 14, 14' weisen jeweils eine Flüssigkristallebene 32 auf. Die Flüssigkristallebene 32 ist von einer transluzenten Flüssigkristallebene gebildet. Die Flüssigkristallebene 32 weist eine Flüssigkristallschicht 34 auf. In der Flüssigkristallschicht 34 befindet sich eine Vielzahl von Kristallmolekülen sowie Abstandshalter. Auf beiden Seiten der Flüssigkristallschicht 34 ist jeweils eine Polyimid-Schicht 35, 35' angeordnet. Die Polyimid-Schichten 35, 35' dienen insbesondere einer Ausrichtung der Kristallmoleküle. Auf den der Flüssigkristallschicht 34 abgewandten Seiten der Polyimid-Schichten 35, 35' ist jeweils eine Elektrodenschicht 36, 36' angeordnet. Die Elektrodenschichten 36, 36' sind jeweils von einer transparenten Indiumzinnoxid-Schicht gebildet. Ferner befindet sich auf beiden Seiten der Flüssigkristallebene 32 der Flüssigkristallzellen 14, 14' jeweils eine Polarisationsschicht 37, 37'. Die Polarisationsschichten 37, 37' dienen jeweils zu einer Polarisation einfallenden Lichts. Auf den der Flüssigkristallebene 32 abgewandten Seiten der Polarisationsschichten 37, 37' ist jeweils eine Scheibe 38, 38' angeordnet. Die Scheiben 38, 38' bestehen aus Kunststoff. Die Scheiben 38, 38' bestehen aus Polykarbonat. Auf einer Außenseite der Scheiben 38a, 38a' ist jeweils eine Antireflexionsschicht 39, 39' sowie eine Hartschicht 40, 40' aufgebracht. Die Flüssigkristallzellen 14, 14' des optischen Sonnenschutzfilters 12 sind jeweils zumindest teilweise gebogen ausgebildet. Die Flüssigkristallzellen 14, 14' des optischen Sonnenschutzfilters 12 sind jeweils über eine gesamte Erstreckung gebogen ausgebildet (Fig. 4).

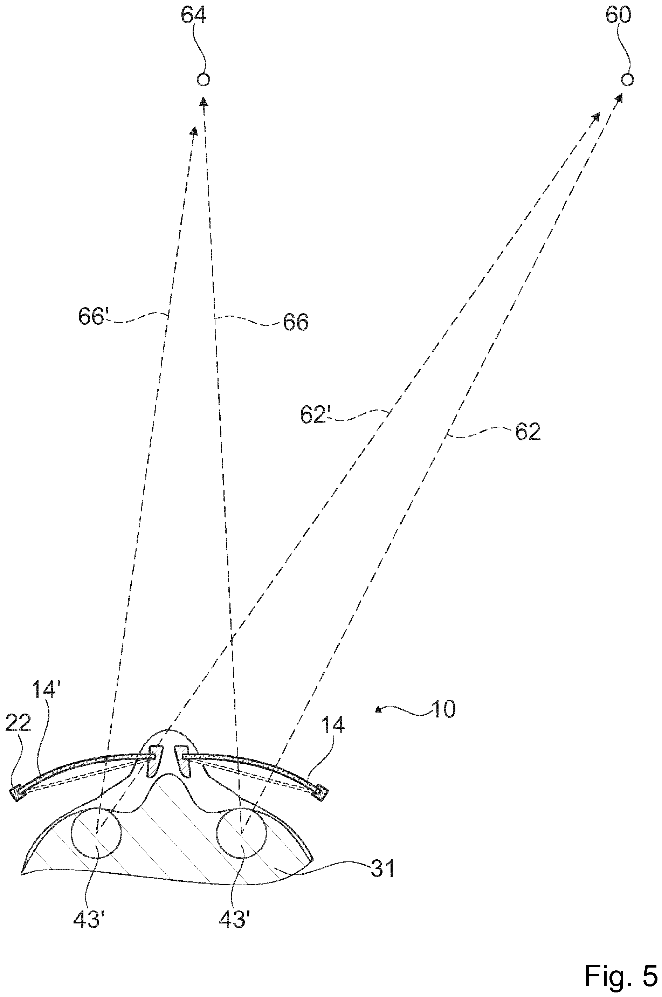

Figur 5 zeigt die Sonnenschutzvorrichtung 10 und den Benutzer 31 mit verschiedenen Blickwinkeln des Benutzers 31 in einer horizontalen Ebene betrachtet. Es ist ein erster Blickwinkel des Benutzers 31 auf einen ersten Punkt 60 dargestellt. Der erste Punkt 60 liegt in einem Randbereich des Sichtfelds des Benutzers 31. Eine Blickrichtung 62 des ersten Auges 43 des Benutzers 31 auf den ersten Punkt 60 fällt mit einem Winkel von 83° durch die gebogene Flüssigkristallzelle 14. Eine Blickrichtung 62' des zweiten Auges 43' des Benutzers 31 auf den ersten Punkt 60 fällt mit einem Winkel von 126° durch die gebogene Flüssigkristallzelle 14'. Eine Differenz zwischen den beiden Blickrichtungen 62, 62' der beiden Augen 43, 43' des Benutzers 31 beträgt daher 43°. Bei derselben Anordnung würde die Differenz bei geraden Flüssigkristallzellen ca. 63° betragen. Ferner ist ein zweiter Blickwinkel des Benutzers 31 auf einen zweiten Punkt 64 dargestellt. Der zweite Punkt 64 liegt in einem Zentrum des Blickfelds des Benutzers 31. Eine Blickrichtung 66 des ersten Auges 43 des Benutzers 31 auf den zweiten Punkt 64 fällt mit einem Winkel von 103° durch die gebogene Flüssigkristallzelle 14. Eine Blickrichtung 66' des zweiten Auges 43' des Benutzers 31 auf den zweiten Punkt 64 fällt mit einem Winkel von 107° durch die gebogene Flüssigkristallzelle 14'. Eine Differenz zwischen den beiden Blickrichtungen 66, 66' der beiden Augen 43, 43' des Benutzers 31 beträgt daher 4°. Bei derselben Anordnung würde die Differenz bei geraden Flüssigkristallzellen ebenfalls ca. 4° betragen. Durch die gebogene Ausgestaltung der Flüssigkristallzellen 14, 14' kann daher insbesondere auch in Randbereichen des Sichtfelds des Benutzers 31 ein vorteilhaft gleichmäßiges Bild erzeugt werden. Es können insbesondere störende Unterschiede zwischen den beiden Augen 43, 43' des Benutzers 31 erreicht werden.

Des Weiteren weist die Sonnenschutzvorrichtung 10 eine Sensoreinheit 16 auf. Die Sensoreinheit 16 ist zu einer Erfassung einer Sonneneinstrahlung vorgesehen ist. Die Sensoreinheit 16 ist in dem Brillenrahmen 22 angeordnet. Die Sensoreinheit 16 ist in dem Grundrahmen 26 des Brillenrahmens 22 zwischen den Ausnehmungen für die Flüssigkristallzellen 14, 14' angeordnet. Die Sensoreinheit 16 ist in dem Grundrahmen 26 des Brillenrahmens 22 oberhalb des Nasenausschnitts 30 angeordnet. Die Sensoreinheit 16 ist zu einer Vorderseite der Sonnenschutzvorrichtung 10 hin unverdeckt. Die Sensoreinheit 16 weist eine Photodiode 24 auf. Die Sensoreinheit 16 weist eine Photozelle auf. Grundsätzlich wäre jedoch auch denkbar, dass die Sensoreinheit 16 eine Solarzelle aufweist. Die Sensoreinheit 16 weist eine von einer Photozelle gebildete Photodiode 24 auf. Die Sensoreinheit 16 ist von der Photodiode 24 gebildet.

Ferner weist die Sonnenschutzvorrichtung 10 eine Steuer- und Regeleinheit 18 auf. Die Steuer- und Regeleinheit 18 ist dazu vorgesehen, abhängig von einer Sonneneinstrahlung eine Durchlässigkeit des optischen Sonnenschutzfilters 12 zu steuern. Die Steuer- und Regeleinheit 18 ist dazu nicht weiter sichtbar mit der Sensoreinheit 16 verbunden. Ferner ist die Steuer- und Regeleinheit 18 nicht weiter sichtbar mit den Flüssigkristallzellen 14, 14' des optischen Sonnenschutzfilters 12 verbunden. Die Steuer- und Regeleinheit 18 ist nicht weiter sichtbar elektrisch mit den Elektrodenschichten 36, 36' der Flüssigkristallzellen 14, 14' verbunden. Die Steuer- und Regeleinheit 18 ist in dem Brillenrahmen 22 angeordnet. Die Steuer- und Regeleinheit 18 ist in dem Grundrahmen 26 des Brillenrahmens 22 zwischen den Ausnehmungen für die Flüssigkristallzellen 14, 14' angeordnet. Die Steuer- und Regeleinheit 18 ist in dem Grundrahmen 26 des Brillenrahmens 22 oberhalb des Nasenausschnitts 30 angeordnet. Grundsätzlich wäre jedoch auch eine andere, einem Fachmann als sinnvoll erscheinende Anordnung der Steuer- und Regeleinheit 18 denkbar. Die Steuer- und Regeleinheit 18 befindet sich ständig in einem Betrieb und kann insbesondere nicht deaktiviert werden. Die Steuer- und Regeleinheit 18 schaltet sich ab einer definierten an der Sensoreinheit 16 anliegenden Spannung ein und beginnt zu schwingen. Grundsätzlich wäre jedoch auch denkbar, dass die Steuer- und Regeleinheit 18 mittels eines nicht weiter sichtbaren Schalters in einen Betrieb versetzt werden kann.

Die Steuer- und Regeleinheit 18 ist in einem Betriebszustand dazu vorgesehen, die Flüssigkristallzellen 14, 14' des optischen Sonnenschutzfilters 12 zu einer Erzeugung eines für einen Benutzer 31 definierten Durchlässigkeitsverlaufs 20 mit zumindest zwei differierenden Durchlässigkeiten zu steuern. Die Steuer- und Regeleinheit 18 ist bei Sonneneinstrahlung dazu vorgesehen, die Flüssigkristallzellen 14, 14' des optischen Sonnenschutzfilters 12 zu einer Erzeugung eines aus einer Blickrichtung des Benutzers 31 definierten Durchlässigkeitsverlaufs 20 mit zumindest zwei differierenden Durchlässigkeiten zu steuern. Die Steuer- und Regeleinheit 18 ist bei Sonneneinstrahlung dazu vorgesehen, die Flüssigkristallzellen 14, 14' des optischen Sonnenschutzfilters 12 zu einer Erzeugung eines aus einer Blickrichtung des Benutzers 31 definierten, kontinuierlichen Durchlässigkeitsverlaufs 20 mit zumindest zwei differierenden Durchlässigkeiten zu steuern. Bei dem Durchlässigkeitsverlauf 20 ist für einen Benutzer 31 eine geringere Durchlässigkeit der Flüssigkristallzellen 14, 14' des optischen Sonnenschutzfilters 12 oberhalb einer höheren Durchlässigkeit angeordnet. Der Durchlässigkeitsverlauf 20 erstreckt sich daher für einen Benutzer 31 von oben nach unten, wobei eine Durchlässigkeit von oben nach unten zunimmt. Ferner erstreckt sich der Durchlässigkeitsverlauf 20 aus einer Blickrichtung des Benutzers 31 parallel zu einer Haupterstreckungsebene 42 der jeweiligen Flüssigkristallzelle 14, 14'. Die Haupterstreckungsebene 42 der Flüssigkristallzellen 14, 14' weist bei einer aufrechten Haltung des Benutzers 21 einen Winkel von 12° gegenüber einer Senkrechten 41 auf. Die Steuer- und Regeleinheit 18 ist in einem Betriebszustand dazu vorgesehen, die Flüssigkristallzellen 14, 14' des optischen Sonnenschutzfilters 12 zu einem Halten des für einen Benutzer 31 definierten Durchlässigkeitsverlaufs 20 zu steuer. Die Steuer- und Regeleinheit 18 ist dazu vorgesehen, bei gleichbleibender Sonneneinstrahlung relativ zu der Sonnenschutzvorrichtung 10a die Flüssigkristallzellen 14, 14' des optischen Sonnenschutzfilters 12 zu einem Halten des aus einer Blickrichtung des Benutzers 31 definierten Durchlässigkeitsverlaufs 20 anzusteuern. Des Weiteren ist die Steuer- und Regeleinheit 18 in einem Betriebszustand dazu vorgesehen, eine Stärke sowie eine Erstreckung des Durchlässigkeitsverlaufs 20 der Flüssigkristallzellen 14, 14' des optischen Sonnenschutzfilters 12 für einen Benutzer 31 abhängig von einer Sonneneinstrahlung einzustellen. Die Steuer- und Regeleinheit 18 ist dabei dazu vorgesehen, eine Erstreckung des Durchlässigkeitsverlaufs 20 der Flüssigkristallzellen 14, 14' des optischen Sonnenschutzfilters 12 aus einer Blickrichtung des Benutzers 31 abhängig von einer Sonneneinstrahlung einzustellen, wobei damit eine durchschnittliche Durchlässigkeit über die Fläche der Flüssigkristallzellen 14, 14' verändert wird. Mit zunehmender Sonneneinstrahlung verringert sich dabei aus einer Blickrichtung des Benutzers 31 eine durchschnittliche Durchlässigkeit über die Fläche der Flüssigkristallzellen 14, 14'. Zusätzlich ist die Steuer- und Regeleinheit 18 dazu vorgesehen, eine Erstreckung des Durchlässigkeitsverlaufs 20 der Flüssigkristallzellen 14, 14' des optischen Sonnenschutzfilters 12 für den Benutzer 31 abhängig von einer Benutzereingabe einzustellen. Die Steuer- und Regeleinheit 18 ist dazu vorgesehen, eine Stärke und Erstreckung des Durchlässigkeitsverlaufs 20 der Flüssigkristallzellen 14, 14' des optischen Sonnenschutzfilters 12 für den Benutzer 31 abhängig von einer benutzereinstellbaren Stufe zur Anpassung des Durchlässigkeitsverlaufs 20 einzustellen. Eine Eingabe der benutzereinstellbaren Stufe erfolgt über eine nicht weiter sichtbare Eingabeeinheit. Die benutzereinstellbaren Stufe definiert dabei einen Korrekturfaktor, des für den Benutzer definierten Durchlässigkeitsverlaufs 20 relativ zu der Sonneneinstrahlung. Der Durchlässigkeitsverlauf 20 erstreckt sich aus einer Blickrichtung des Benutzers 31 mit zunehmender Sonneneinstrahlung immer weiter von oben nach unten in die Flüssigkristallzellen 14, 14'. Der Durchlässigkeitsverlauf 20 ist dabei für einen Benutzer 31 in einem getragenen Zustand der Sonnenschutzvorrichtung 10 sichtbar und wird durch die optischen Eigenschaften der Flüssigkristalle der Flüssigkristallzellen 14, 14' selbst erzeugt. Der definierte Durchlässigkeitsverlauf 20 entsteht dabei durch die Winkelabhängigkeit der Flüssigkristallzellen 14, 14', also eine Beeinflussung der Transmission der Flüssigkristallzellen 14, 14' abhängig von einem Blickwinkel der Augen 43, 43' des Benutzers 31 auf die Kristallmoleküle der Flüssigkristallschicht 34 sowie abhängig von der Orientierung der Kristallmoleküle. Mit zunehmender Abwinklung der Blickrichtung bzw. des Blickwinkels gegenüber den Kristallmolekülen der Flüssigkristallschicht 34 der Flüssigkristallzelle 14, 14' sinkt die Transmission der Flüssigkristallzelle und eine Absorption wird erhöht.

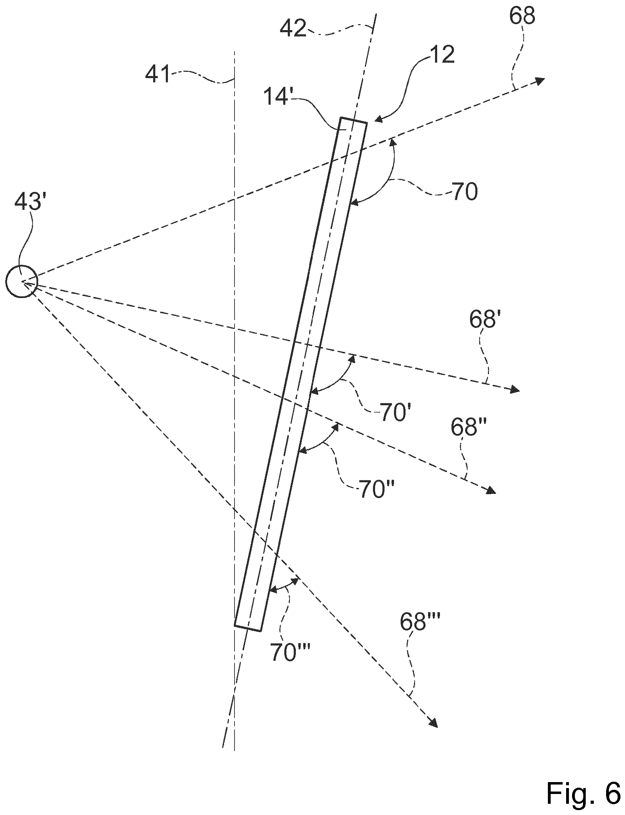

Figur 6 zeigt die Sonnenschutzvorrichtung 10 und den Benutzer 31 mit verschiedenen Blickwinkeln des Benutzers 31 durch die Flüssigkristallzelle 14' in einer vertikalen Ebene betrachtet. Es ist ein erster Blickwinkel des Benutzers 31 mit einer ersten Blickrichtung 68 dargestellt. Die erste Blickrichtung 68 fällt in einem oberen Bereich der Flüssigkristallzelle 14' durch die Flüssigkristallzelle 14'. Die erste Blickrichtung 68 fällt mit einem ersten Durchschauwinkel 70 durch die Flüssigkristallzelle 14. Der erste Durchschauwinkel 70 beträgt annähernd 123°. Ferner ist ein zweiter Blickwinkel des Benutzers 31 mit einer zweiten Blickrichtung 68' dargestellt. Die zweite Blickrichtung 68' fällt unterhalb der ersten Blickrichtung 68 in einem mittleren Bereich der Flüssigkristallzelle 14' durch die Flüssigkristallzelle 14'. Die zweite Blickrichtung 68' fällt mit einem zweiten Durchschauwinkel 70' durch die Flüssigkristallzelle 14. Der zweite Durchschauwinkel 70' beträgt annähernd 90°. Des Weiteren ist ein dritter Blickwinkel des Benutzers 31 mit einer dritten Blickrichtung 68" dargestellt. Die dritte Blickrichtung 68" fällt unterhalb der zweiten Blickrichtung 68' in einem mittleren Bereich der Flüssigkristallzelle 14' durch die Flüssigkristallzelle 14'. Die dritte Blickrichtung 68" fällt mit einem dritten Durchschauwinkel 70" durch die Flüssigkristallzelle 14. Der dritte Durchschauwinkel 70" beträgt annähernd 77°. Zudem ist ein vierter Blickwinkel des Benutzers 31 mit einer vierten Blickrichtung 68‴ dargestellt. Die vierte Blickrichtung 68‴ fällt unterhalb der dritten Blickrichtung 68" in einem unteren Bereich der Flüssigkristallzelle 14' durch die Flüssigkristallzelle 14'. Die vierte Blickrichtung 68‴ fällt mit einem vierten Durchschauwinkel 70‴ durch die Flüssigkristallzelle 14. Der vierte Durchschauwinkel 70‴ beträgt annähernd 55°. Mit zunehmender Senkung des Blickes sinkt daher auch ein Durchschauwinkel des Benutzers 31 durch die Flüssigkristallzelle 14'. Aufgrund der Blickwinkelabhängigkeit der Flüssigkristallzellen 14, 14' steigt damit zumindest während eines Betriebs mit abnehmender Abwinklung der Blickrichtung bzw. des Blickwinkels gegenüber den Kristallmolekülen der Flüssigkristallschicht 34 der Flüssigkristallzellen 14, 14' die Transmission der Flüssigkristallzelle und eine Absorption wird verringert. Eine Transmission der vierten Blickrichtung 68‴ des Benutzers 31 ist daher während eines Betriebs der Sonnenschutzvorrichtung 10 wesentlich höher, als eine Transmission der ersten Blickrichtung 68 des Benutzers 31. Es entsteht für den Benutzer 31 ein definierter Durchlässigkeitsverlauf 20.

Die Steuer- und Regeleinheit 18 ist in einem Betriebszustand dazu vorgesehen, eine Ansteuerspannung V_{A} zu einer Ansteuerung der Flüssigkristallzellen 14, 14' des optischen Sonnenschutzfilters 12 auf einen Wert unterhalb einer Sättigungsfeldstärke der Flüssigkristallzellen 14, 14' zu steuern. Die Steuer- und Regeleinheit 18 ist zu einer Erzeugung des Durchlässigkeitsverlaufs 20 dazu vorgesehen, eine Ansteuerspannung V_{A} zu einer Ansteuerung der Flüssigkristallzellen 14, 14' des optischen Sonnenschutzfilters 12 auf einen Wert unterhalb einer zu einer Bereitstellung einer Sättigungsfeldstärke der Flüssigkristallzellen 14, 14' benötigten Sättigungsspannung V_{S} zu steuern. Die Sättigungsspannung V_{S} zu einer Bereitstellung einer Sättigungsfeldstärke der Flüssigkristallzellen 14, 14' bildet eine Sättigungsspannung. Die Flüssigkristallzellen 14, 14' des optischen Sonnenschutzfilters 12 werden grundsätzlich mit einer Ansteuerspannung V_{A} angesteuert, welche geringer als die Sättigungsspannung V_{S} ist. Mit zunehmender Sonneneinstrahlung wird die Ansteuerspannung V_{A} erhöht. Die Ansteuerspannung V_{A} ist dabei die Spannung, welche an den Elektrodenschichten 36, 36' der Flüssigkristallzellen 14, 14' zu einer Erzeugung eines elektrischen Felds zu einer Ausrichtung der Flüssigkristallschicht 43 der Flüssigkristallzellen 14, 14' angelegt wird. Bei einem Anlegen einer Ansteuerspannung V_{A}, welche unterhalb einer zu der Bereitstellung der Sättigungsfeldstärke der Flüssigkristallzellen 14, 14' benötigten Sättigungsspannung V_{S} liegt, werden die Kristallmoleküle der Flüssigkristallschicht 43 der Flüssigkristallzellen 14, 14' lediglich teilweise ausgelenkt, sodass eine Blickwinkelabhängigkeit der Flüssigkristallzellen 14, 14' auftritt. Für den Benutzer 31 entsteht der Durchlässigkeitsverlauf 20 in den Flüssigkristallzellen 14, 14'.

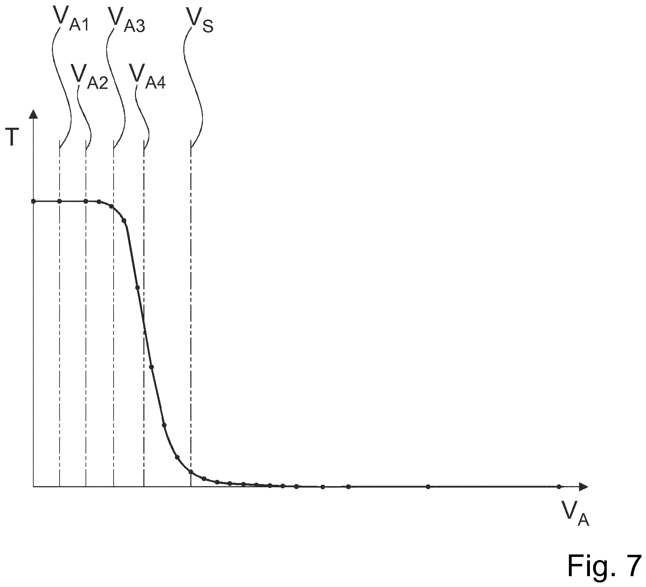

Figur 7 zeigt ein Diagramm einer Transmission T der Flüssigkristallzellen 14, 14' des Sonnenschutzfilters 12 abhängig von einer Ansteuerspannung V_{A}. Dabei ist auf einer vertikalen y-Achse die Transmission T aufgetragen, wobei auf der horizontalen x-Achse die Ansteuerspannung V_{A} aufgetragen ist. Das Diagramm stellt dabei die durchschnittliche Transmission T der Flüssigkristallzellen 14, 14' über die Fläche in Prozent abhängig von einer Ansteuerspannung V_{A} dar. Ferner ist in dem Diagramm die Sättigungsspannung V_{S} dargestellt. Bei Ansteuerspannungen V_{A}, welche größer null und geringer als die Sättigungsspannung V_{S} sind, liegt aus der Blickrichtung des Benutzers ein Durchlässigkeitsverlauf 20 vor.

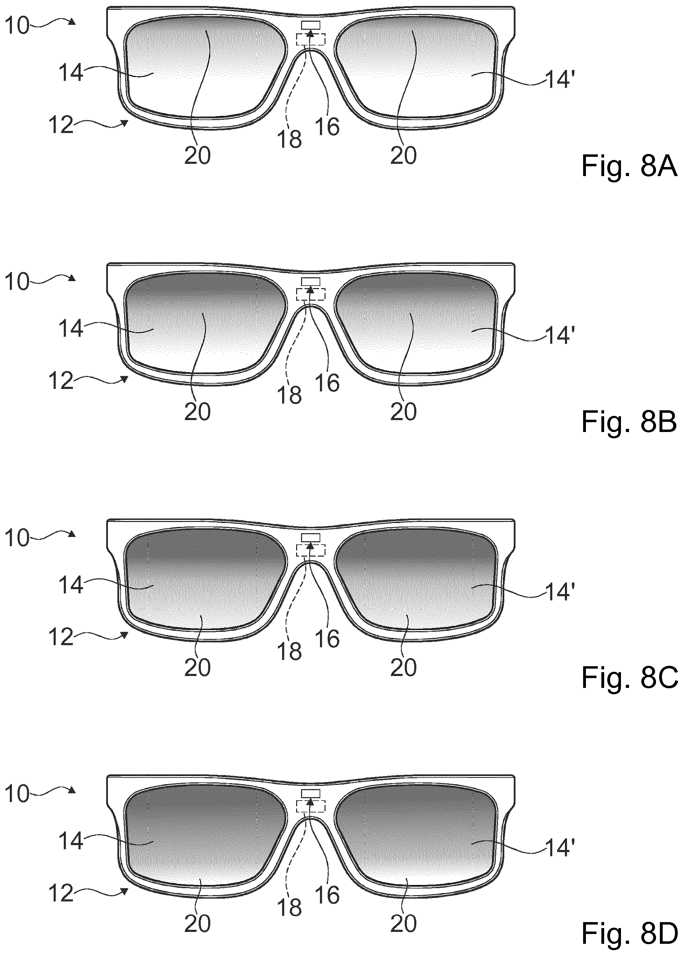

In den Figuren 8A, 8B, 8C und 8D sind jeweils beispielhaft vier verschiedene Zustände der Sonnenschutzvorrichtung 10 dargestellt. Die Figuren 8A, 8B, 8C und 8D zeigen dabei jeweils den Durchlässigkeitsverlauf 20 der Flüssigkristallzellen 14, 14' des optischen Sonnenschutzfilters 12 in den jeweiligen Zuständen, wobei in dem Diagramm der Figur 4 jeweils die zugehörige Ansteuerspannung V_{A}, welche in dem entsprechenden Zustand an den Elektrodenschichten 36, 36' der Flüssigkristallzellen 14, 14' anliegt, dargestellt ist. Die Ansteuerspannung V_{A1} ist dabei dem ersten Zustand zugeordnet, welcher in der Figur 8A dargestellt ist. Die Ansteuerspannung V_{A2} ist dabei dem zweiten Zustand zugeordnet, welcher in der Figur 8B dargestellt ist. Die Ansteuerspannung V_{A3} ist dabei dem dritten Zustand zugeordnet, welcher in der Figur 8C dargestellt ist. Die Ansteuerspannung V_{A4} ist dabei dem vierten Zustand zugeordnet, welcher in der Figur 8D dargestellt ist. Bei dem ersten Zustand liegt dabei gegenüber den anderen Zuständen eine geringe Sonneneinstrahlung vor. Bei dem vierten Zustand liegt gegenüber den anderen Zuständen eine hohe Sonneneinstrahlung vor. Die Ansteuerspannung V_{A} wird mit zunehmender Sonneneinstrahlung erhöht.

Die Sensoreinheit 16 ist in einem Betriebszustand zu einer zumindest teilweisen Bereitstellung einer Energie zu einer Ansteuerung der Flüssigkristallzellen 14, 14' des optischen Sonnenschutzfilters 12 vorgesehen. Die Sensoreinheit 16 ist während eines Betriebs der Sonnenschutzvorrichtung 10 zu einer vollständigen Bereitstellung einer Energie zu einer Ansteuerung der Flüssigkristallzellen 14, 14' des optischen Sonnenschutzfilters 12 vorgesehen. Die Sensoreinheit 16 ist während eines Betriebs der Sonnenschutzvorrichtung 10 zu einer vollständigen Gewinnung einer zu einer Ansteuerung der zumindest einen Flüssigkristallzellen 14, 14' des optischen Sonnenschutzfilters 12 benötigten Energie vorgesehen. Die Sensoreinheit 16 stellt die Ansteuerspannung V_{A} bereit. Ferner ist die Sonnenschutzvorrichtung 10 daher batterielos ausgebildet.

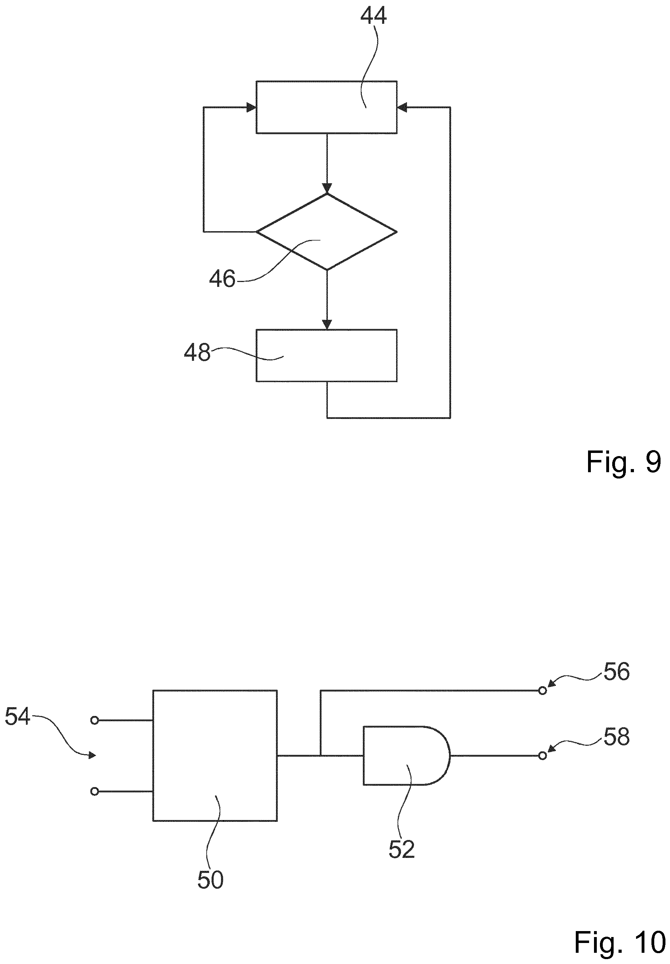

Figur 9 zeigt ein Ablaufdiagramm eines Verfahrens zu einem Betrieb einer Sonnenschutzvorrichtung 10. Bei dem Verfahren werden Flüssigkristallzellen 14, 14' des optischen Sonnenschutzfilters 12 zu einer Erzeugung eines für den Benutzer definierten Durchlässigkeitsverlaufs 20 angesteuert. Die Flüssigkristallzellen 14, 14' des optischen Sonnenschutzfilters 12 werden zu einer Erzeugung eines für den Benutzer definierten Durchlässigkeitsverlaufs 20 abhängig von einer Sonneneinstrahlung angesteuert. Mittels der Sensoreinheit 16 wird in einem Schritt 44 eine Sonneneinstrahlung erfasst. Anschließend wird in einer Verzweigung 46 ein Wert der von der Sensoreinheit 16 generierten Spannung überwacht. Wird ein Schwellwert nicht überschritten, wird der Schritt 44 wiederholt. Übersteigt der Wert der von der Sensoreinheit 16 generierten Spannung einen bestimmten Wert, wird in einem weiteren Schritt 48 mittels einer astabilen Kippstufe 50 eine Schwingung in der Höhe der Sensorspannung erzeugt, so dass durch ein nachgeschaltetes NAND-Gatter 52 die Wechselspannungen für das Ansteuern der Flüssigkristallzellen 14, 14' generiert werden können. Zusätzlich werden die Flüssigkristallzellen 14, 14' des optischen Sonnenschutzfilters 12 zu einer Erzeugung des für den Benutzer definierten Durchlässigkeitsverlaufs 20 abhängig von einer Benutzereingabe angesteuert. Die Flüssigkristallzellen 14, 14' des optischen Sonnenschutzfilters 12 werden zu einer Erzeugung des für den Benutzer definierten Durchlässigkeitsverlaufs 20 abhängig von einer Benutzereinstellbaren Stufe zur Anpassung des Durchlässigkeitsverlaufs angesteuert.

Figur 10 zeigt ein schematisches Schaltbild der Steuer- und Regeleinheit 18. Auf einer Eingangsseite 54 der Steuer- und Regeleinheit 18 ist die Sensoreinheit 16 angeordnet. Auf den beiden Ausgangsseiten 56, 58 der Steuer- und Regeleinheit 18 sind die Elektrodenschichten 36, 36' der Flüssigkristallzellen 14, 14' angeordnet.

## Patentansprüche

1. Sonnenschutzvorrichtung, insbesondere Sonnenbrille, mit zumindest einem optischen Sonnenschutzfilter (12), der zumindest eine Flüssigkristallzelle (14, 14') aufweist, mit zumindest einer Sensoreinheit (16), welche zu einer Erfassung einer Sonneneinstrahlung vorgesehen ist, und mit zumindest einer Steuer- und/oder Regeleinheit (18), welche dazu vorgesehen ist, abhängig von einer Sonneneinstrahlung eine Durchlässigkeit des optischen Sonnenschutzfilters (12) zu steuern und/oder zu regeln, wobei die Steuer- und/oder Regeleinheit (18) in zumindest einem Betriebszustand dazu vorgesehen ist, die zumindest eine Flüssigkristallzelle (14, 14') des optischen Sonnenschutzfilters (12) zu einer Erzeugung eines für einen Benutzer (31) definierten Durchlässigkeitsverlaufs (20) mit zumindest zwei differierenden Durchlässigkeiten anzusteuern, wobei der optische Sonnenschutzfilter (12) zwei Flüssigkristallzellen (14, 14'), die jeweils einem Auge des Benutzers (31) zugeordnet sind, oder lediglich eine Flüssigkristallzelle (14, 14') für beide Augen des Benutzers (31) aufweist, wobei die zumindest eine Flüssigkristallzelle (14, 14') eine in der Transmission T schaltbare Flüssigkristallebene (32) aufweist, wobei der definierte Durchlässigkeitsverlauf (20) ein aus einer Blickrichtung des Benutzers (31), insbesondere des Trägers, sichtbarer Verlauf einer Durchlässigkeit der Flüssigkristallzelle (14, 14') ist, welcher zu einem festen Zeitpunkt zumindest zwei differierende Durchlässigkeiten aufweist, wobei der definierte Durchlässigkeitsverlauf (20) durch die Winkelabhängigkeit der Flüssigkristallzelle (14, 14'), also eine Beeinflussung der Transmission der Flüssigkristallzelle (14, 14') abhängig von einem Blickwinkel des Auges des Benutzers (31) auf die Kristallmoleküle sowie abhängig von der Orientierung der Kristallmoleküle, entsteht, wobei mit zunehmender Senkung des Blickes auch ein Durchschauwinkel des Benutzers (31) durch die Flüssigkristallzelle (14, 14') sinkt, wobei mit abnehmender Abwinklung der Blickrichtung bzw. des Blickwinkels gegenüber den Kristallmolekülen der Flüssigkristallschicht (34) der Flüssigkristallzelle (14, 14') die Transmission der Flüssigkristallzelle (14, 14') steigt und eine Absorption verringert wird, wobei die Blickwinkelabhängigkeit der Flüssigkristallzelle (14, 14') mittels eines Anlegens einer Ansteuerspannung (V_{A}), welche durch die Steuer- und Regeleinheit (18) gesteuert wird, welche unterhalb einer zu der Bereitstellung der Sättigungsfeldstärke der Flüssigkristallzellen (14, 14') benötigten Sättigungsspannung (V_{S}) liegt, wodurch die Kristallmoleküle einer Flüssigkristallschicht (43) der Flüssigkristallzellen (14, 14') lediglich teilweise ausgelenkt werden, auftritt, und wobei die Steuer- und/oder Regeleinheit (18) in zumindest einem Betriebszustand dazu vorgesehen ist, die zumindest eine Flüssigkristallzelle (14, 14') des optischen Sonnenschutzfilters (12) zu einem Halten des für den Benutzer (31) definierten Durchlässigkeitsverlaufs (20), also zu einem Beibehalten des zu einem Zeitpunkt vorliegenden aus einer Blickrichtung des Benutzers definierten Durchlässigkeitsverlaufs über einen unbegrenzten Zeitraum hinweg, anzusteuern, wobei auf beiden Seiten der Flüssigkristallschicht (34) jeweils eine Polyimid-Schicht (35, 35') angeordnet ist, wobei auf den der Flüssigkristallschicht (34) abgewandten Seiten der Polyimid-Schichten (35, 35') jeweils eine Elektrodenschicht (36, 36') angeordnet ist, wobei die Ansteuerspannung V_{A} dabei die Spannung ist, welche an den Elektrodenschichten (36, 36') der Flüssigkristallzellen (14, 14') zu einer Erzeugung eines elektrischen Felds zu einer Ausrichtung der Flüssigkristallschicht (43) der Flüssigkristallzellen (14, 14') angelegt wird, wobei die Steuer- und/oder Regeleinheit (18) in zumindest einem Betriebszustand dazu vorgesehen ist, eine Stärke und/oder Erstreckung des Durchlässigkeitsverlaufs (20) der Flüssigkristallzellen (14, 14') des optischen Sonnenschutzfilters (12) für den Benutzer (31) abhängig von einer Sonneneinstrahlung einzustellen, wobei die Steuer- und/oder Regeleinheit (18) in zumindest einem Betriebszustand dazu vorgesehen ist, eine Erstreckung des Durchlässigkeitsverlaufs der Flüssigkristallzellen (14, 14') des optischen Sonnenschutzfilters (12) aus einer Blickrichtung des Benutzers (31) abhängig von einer Sonneneinstrahlung einzustellen, wobei damit für einen Benutzer eine durchschnittliche Durchlässigkeit über die Fläche der Flüssigkristallzellen (14, 14') verändert wird, wobei die zumindest eine Sensoreinheit (16) während eines Betriebs der Sonnenschutzvorrichtung zu einer vollständigen Bereitstellung einer Energie zu einer Ansteuerung der zumindest einen Flüssigkristallzelle (14, 14') des optischen Sonnenschutzfilters (12) vorgesehen ist, **dadurch gekennzeichnet, dass** die Sonnenschutzvorrichtung batterielos ausgebildet ist.

2. Sonnenschutzvorrichtung nach Anspruch 1,
**dadurch gekennzeichnet, dass**
für den Benutzer (31) eine geringere Durchlässigkeit der zumindest einen Flüssigkristallzelle (14, 14') bei dem Durchlässigkeitsverlauf (20) oberhalb einer höheren Durchlässigkeit angeordnet ist.

3. Sonnenschutzvorrichtung nach einem der vorhergehenden Ansprüche,
**dadurch gekennzeichnet, dass**
die Steuer- und/oder Regeleinheit (18) in zumindest einem Betriebszustand dazu vorgesehen ist, eine Ansteuerspannung (V_{A}) zu einer Ansteuerung der zumindest einen Flüssigkristallzelle (14, 14') des optischen Sonnenschutzfilters (12) auf einen Wert unterhalb einer Sättigungsfeldstärke der zumindest einen Flüssigkristallzelle (14, 14') zu steuern und/oder zu regeln.

4. Sonnenschutzvorrichtung nach einem der vorhergehenden Ansprüche,
**dadurch gekennzeichnet, dass**
die zumindest eine Flüssigkristallzelle (14, 14') des optischen Sonnenschutzfilters (12) zumindest teilweise gebogen ausgebildet ist.

5. Sonnenschutzvorrichtung nach einem der vorhergehenden Ansprüche,
**dadurch gekennzeichnet, dass**
die zumindest eine Flüssigkristallzelle (14, 14') des optischen Sonnenschutzfilters (12) von einer Kunststoff-Flüssigkristallzelle gebildet ist.

6. Sonnenschutzvorrichtung nach einem der vorhergehenden Ansprüche,
**gekennzeichnet durch**
einen Brillenrahmen (22), welcher zu einer Aufnahme des optischen Sonnenschutzfilters (12) vorgesehen ist.

7. Sonnenschutzvorrichtung nach einem der vorhergehenden Ansprüche,
**dadurch gekennzeichnet, dass**
die zumindest eine Sensoreinheit (16) in zumindest einem Betriebszustand zu einer zumindest teilweisen Bereitstellung einer Energie zu einer Ansteuerung der zumindest einen Flüssigkristallzelle (14, 14') des optischen Sonnenschutzfilters (12) vorgesehen ist.

8. Sonnenschutzvorrichtung nach einem der vorhergehenden Ansprüche,
**dadurch gekennzeichnet, dass**
die zumindest eine Sensoreinheit (16) zumindest eine Photodiode (24) aufweist.

9. Verfahren zu einem Betrieb einer Sonnenschutzvorrichtung (10) nach einem der vorhergehenden Ansprüche.

10. Verfahren nach Anspruch 9,
**dadurch gekennzeichnet, dass**
die zumindest eine Flüssigkristallzelle (14, 14') des optischen Sonnenschutzfilters (12) zu einer Erzeugung eines für einen Benutzer (31) definierten Durchlässigkeitsverlaufs (20) angesteuert und/oder geregelt wird.

11. Verfahren nach Anspruch 9 oder 10,
**dadurch gekennzeichnet, dass**
die zumindest eine Flüssigkristallzelle (14, 14') des optischen Sonnenschutzfilters (12) zu einer Erzeugung eines für einen Benutzer (31) definierten Durchlässigkeitsverlaufs (20) abhängig von einer Sonneneinstrahlung angesteuert und/oder geregelt wird.

12. Verfahren nach einem der Ansprüche 9 bis 11,
**dadurch gekennzeichnet, dass**
die zumindest eine Flüssigkristallzelle (14, 14') des optischen Sonnenschutzfilters (12) zu einer Erzeugung eines für einen Benutzer (31) definierten Durchlässigkeitsverlaufs (20) abhängig von zumindest einer Benutzereingabe angesteuert und/oder geregelt wird.

13. Verfahren nach Anspruch 12,
**dadurch gekennzeichnet, dass**
die zumindest eine Flüssigkristallzelle (14, 14') des optischen Sonnenschutzfilters (12) zu einer Erzeugung eines für einen Benutzer (31) definierten Durchlässigkeitsverlaufs (20) abhängig von einer benutzereinstellbaren Stufe zur Anpassung des Durchlässigkeitsverlaufs (20) angesteuert und/oder geregelt wird, wobei die Stufe über eine Eingabeeinheit auswählbar ist.

## Claims

1. Sun protection device, in particular sun spectacles,
with at least one optical sun protection filter (12) comprising at least one liquid-crystal cell (14, 14'),
with at least one sensor unit (16) configured for capturing a solar irradiation, and with at least one control and/or regulation unit (18) configured for controlling and/or regulating a permeability of the optical sun protection filter (12) depending on a solar irradiation,
wherein the control and/or regulation unit (18) is in at least one operating state configured for actuating the at least one liquid-crystal cell (14, 14') of the optical sun protection filter (12) for generating a permeability gradient (20) which is defined for a user (31) and features at least two differing permeabilities, wherein the optical sun protection filter (12) comprises two liquid-crystal cells (14, 14') which are respectively allocated to an eye of the user (31), or comprises only one liquid-crystal cell (14, 14') for both eyes of the user (31),
wherein the at least one liquid-crystal cell (14, 14') has a liquid-crystal plane (32) that is switchable in the transmittance T,
wherein the defined permeability gradient (20) is a progression of a permeability of the liquid-crystal cell (14, 14') which is visible from a viewing direction of the user (31), in particular the wearer, and which features at least two differing permeabilities at a fix point in time,
wherein the defined permeability gradient (20) results from the angle-dependency of the liquid-crystal cell (14, 14'), i.e. an influence on the transmittance of the liquid-crystal cell (14, 14') depending on a viewing angle of the eye of the user (31) onto the crystal molecules and depending on the orientation of the crystal molecules,
wherein with increasing lowering of the vision a look-through angle of the user (31) through the liquid-crystal cell (14, 14') is also lowered,
wherein with decreasing angling of the viewing direction, respectively the viewing angle, with respect to the crystal molecules of the liquid-crystal layer (34) of the liquid-crystal cell (14, 14'), the transmittance of the liquid-crystal cell (14, 14') increases and absorption is reduced,
wherein the angle-dependency of the liquid-crystal cell (14, 14') occurs as a result of applying an actuation voltage (V_{A}), which is controlled by the control and regulation unit (18) and is below a saturation voltage (V_{S}) required for supplying the saturation field strength of the liquid-crystal cells (14, 14'), such that the crystal molecules of a liquid-crystal layer (34) of the liquid-crystal cells (14, 14') are only partly deflected,
and wherein the control and/or regulation unit (18) is in at least one operating state configured for actuating the at least one liquid-crystal cell (14, 14') of the optical sun protection filter (12) for maintaining the permeability gradient (20) which is defined for the user (31), i. e. for maintaining the permeability gradient which is present at a point in time and is defined from a viewing direction of the user (31), over an infinite period,
wherein on both sides of the liquid-crystal layer (34) respectively one polyimide layer (35, 35') is arranged, wherein on the sides of the polyimide layers (35, 35') facing away from the liquid crystal layer (34), respectively one electrode layer (36, 36') is arranged,
the actuation voltage (V_{A}) being the voltage which is applied to the electrode layers (36, 36') of the liquid-crystal cells (14, 14') for the purpose of generating an electrical field for aligning the liquid-crystal layer (34) of the liquid-crystal cells (14, 14'),
wherein the control and/or regulation unit (18) is configured, in at least one operating state, for adjusting, depending on a solar irradiation, an intensity and/or extension of the permeability gradient (20) of the liquid-crystal cells (14, 14') of the optical sun protection filter (12) for the user (31),
wherein the control and/or regulation unit (18) is configured, in at least one operating state, for adjusting, depending on a solar irradiation, an extension of the permeability gradient (20) of the liquid-crystal cells (14, 14') of the optical sun protection filter (12) from a viewing direction of the user (31), thus changing for a user an average permeability over the area of the liquid-crystal cells (14, 14'), wherein during operation of the sun protection device, the at least one sensor unit (16) is configured to completely supply an energy for actuating the at least one liquid-crystal cell (14, 14') of the optical sun protection filter (12),
**characterized in that**
the sun protection device is embodied battery-free.

2. Sun protection device according to claim 1,
**characterized in that**
for the user (31) a lower permeability of the at least one liquid-crystal cell (14, 14') is arranged below a higher permeability in the permeability gradient (20).

3. Sun protection device according to one of the preceding claims,
**characterized in that**
the control and/or regulation unit (18) is in at least one operating state configured for actuating and/or regulating an actuation voltage (V_{A}), for controlling the at least one liquid-crystal cell (14, 14') of the optical sun protection filter (12), to a value below a saturation field strength of the at least one liquid-crystal cell (14, 14').

4. Sun protection device according to one of the preceding claims,
**characterized in that**
the at least one liquid-crystal cell (14, 14') of the optical sun protection filter (12) is embodied at least partly curved.

5. Sun protection device according to one of the preceding claims,
**characterized in that**
the at least one liquid-crystal cell (14, 14') of the optical sun protection filter (12) is implemented by a plastic liquid-crystal cell.

6. Sun protection device according to one of the preceding claims,
**characterized by**
a spectacle frame (22) which is configured for accommodating the optical sun protection filter (12).

7. Sun protection device according to one of the preceding claims,
**characterized in that**
the at least one sensor unit (16) is in at least one operating state configured for at least partly supplying an energy for an actuation of the at least one liquid-crystal cell (14, 14') of the optical sun protection filter (12).

8. Sun protection device according to one of the preceding claims,
**characterized in that**
the at least one sensor unit (16) comprises at least one photodiode (24).

9. Method for operating a sun protection device (10) according to one of the preceding claims.

10. Method according to claim 9,
**characterized in that**
the at least one liquid-crystal cell (14, 14') of the optical sun protection filter (12) is actuated and/or regulated for generating a permeability gradient (20) defined for a user (31).

11. Method according to claim 9 or 10,
**characterized in that**
the at least one liquid-crystal cell (14, 14') of the optical sun protection filter (12) is actuated and/or regulated depending on a solar irradiation for generating a permeability gradient (20) defined for a user (31).

12. Method according to one of claims 9 to 11,
**characterized in that**
the at least one liquid-crystal cell (14, 14') of the optical sun protection filter (12) is actuated and/or regulated, depending on at least one user input, for generating a permeability gradient (20) defined for a user (31).

13. Method according to claim 15,
**characterized in that**
for the purpose of generating a permeability gradient (20) defined for a user (31), the at least one liquid-crystal cell (14, 14') of the optical sun protection filter (12) is actuated and/or regulated depending on a user-adjustable level for adapting the permeability gradient (20),
wherein the level is selectable via an input unit.

## Revendications

1. Dispositif de protection solaire, en particulier lunettes de soleil,
avec au moins un filtre de protection solaire optique (12) comprenant au moins une cellule à cristaux liquides (14, 14'),
avec au moins une unité captrice (16) prévue pour détecter un rayonnement solaire
et avec au moins une unité de commande et/ou de régulation (18) prévue pour commander et/ou réguler une perméabilité du filtre de protection solaire optique (12) en fonction d'un rayonnement solaire,
l'unité de commande et/ou de régulation (18) étant prévue, en au moins un état de fonctionnement, pour actionner l'au moins une cellule à cristaux liquides (14, 14') du filtre de protection solaire optique (12) pour générer un gradient de perméabilité (20) définie pour un utilisateur (31) ayant au moins deux perméabilités différentes,
le filtre de protection solaire optique (12) comprenant deux cellules à cristaux liquides (14, 14') respectivement associées à un œil de l'utilisateur (31), ou comprenant seulement une cellule à cristaux liquides (14, 14') pour les deux yeux de l'utilisateur (31),
l'au moins une cellule à cristaux liquides (14, 14') comprenant un plan de cristaux liquides (32) qui est commutable dans la transmission T,
où le gradient de perméabilité définie (20) est un gradient, visible d'une direction de vue de l'utilisateur (31), en particulier du porteur, d'une perméabilité de la cellule à cristaux liquides (14, 14') qui présente à un instant fixe au moins deux perméabilités différentes,
où le gradient de perméabilité définie (20) est créée par la dépendance angulaire de la cellule à cristaux liquides (14, 14'), c'est-à-dire une influence sur la transmission de la cellule à cristaux liquides (14, 14') en fonction d'un angle de vue de l'œil de l'utilisateur (31) sur les molécules cristallines ainsi qu'en fonction de l'orientation des molécules cristallines,
où un angle de vue de l'utilisateur (31) à travers la cellule à cristaux liquides (14, 14') diminue également avec une diminution croissante du regard,
où avec un coudage décroissant de la direction de vue et/ou de l'angle de vue par rapport aux molécules cristallines de la couche à cristaux liquides (34) de la cellule à cristaux liquides (14, 14') la transmission de la cellule à cristaux liquides (14, 14') augmente et une absorption est réduite,
où la dépendance de l'angle de vue de la cellule à cristaux liquides (14, 14') se produit moyennant l'application d'un voltage d*actionnement (V_{A}) qui est commandé par l'unité de commande et de régulation (18) et est inférieure à un voltage de saturation (V_{S}) nécessaire pour fournir l'intensité-champ de saturation des cellules à cristaux liquides (14, 14'), de sorte que les molécules cristallines d'une couche à cristaux liquides (34) des cellules à cristaux liquides (14, 14') ne sont que partiellement déviées,
et où l'unité de commande et/ou de régulation (18) est prévue, en au moins un état de fonctionnement, pour actionner l'au moins une cellule à cristaux liquides (14, 14') du filtre de protection solaire optique (12) pour maintenir le gradient de perméabilité (20) qui est définie pour l'utilisateur (31), c'est-à-dire pour maintenir à travers une période infinie le gradient de perméabilité présente à un instant et définie d'une direction de vue de l'utilisateur,
où respectivement une couche à polyimide (35, 35') est disposée sur les deux côtés de la couche à cristaux liquides (34), où respectivement une couche-électrode (36, 36') est disposée sur les côtés des couches de polyimide (35, 35') détournées de la couche à cristaux liquides (34),
où le voltage d'actionnement (V_{A}) est le voltage appliqué aux couches-électrode (36, 36') des cellules à cristaux liquides (14, 14') pour générer un champ électrique pour l'orientation de la couche à cristaux liquides (34) des cellules à cristaux liquides (14, 14'),
où l'unité de commande et/ou de régulation (18) est prévue, en au moins un état de fonctionnement, pour régler pour l'utilisateur (31) une intensité et/ou une étendue de le gradient de perméabilité (20) des cellules à cristaux liquides (14, 14') du filtre de protection solaire optique (12) en fonction d'un rayonnement solaire,
où l'unité de commande et/ou de régulation (18) est prévue, en au moins un état de fonctionnement, pour régler une étendue de le gradient de perméabilité des cellules à cristaux liquides (14, 14') du filtre de protection solaire optique (12) d'une direction de vue de l'utilisateur (31) en fonction d'un rayonnement solaire, où ainsi une perméabilité moyenne est modifiée pour un utilisateur à travers une surface des cellules à cristaux liquides (14, 14*),
où l*au moins une unité captrice (16) est prévue, pendant un fonctionnement du dispositif de protection solaire, pour complètement fournir une énergie pour l'actionnement de l'au moins une cellule à cristaux liquides (14, 14') du filtre de protection solaire optique (12),
**caractérisé en ce que**
le dispositif de protection solaire est réalisé sans batterie.

2. Dispositif de protection solaire selon la revendication 1,
**caractérisé en ce que**
pour l'utilisateur (31), dans le gradient de perméabilité (20) une perméabilité inférieure de l'au moins une cellule à cristaux liquides (14, 14') est disposée au-dessous d'une perméabilité supérieure.

3. Dispositif de protection solaire selon l'une des revendications précédentes,
**caractérisé en ce que**
pour l*actionnement de l'au moins une cellule à cristaux liquides (14, 14') du filtre de protection solaire optique (12), l'unité de commande et/ou de régulation (18) est prévue en au moins un état de fonctionnement pour commander et/ou réguler un voltage d'actionnement (V_{A}) à une valeur inférieure à une intensité-champ de saturation de l'au moins une cellule à cristaux liquides (14, 14').

4. Dispositif de protection solaire selon l'une des revendications précédentes,
**caractérisé en ce que**
l'au moins une cellule à cristaux liquides (14, 14') du filtre de protection solaire optique (12) est réalisée au moins partiellement courbée.

5. Dispositif de protection solaire selon l'une des revendications précédentes,
**caractérisé en ce que**
l'au moins une cellule à cristaux liquides (14, 14') du filtre de protection solaire optique (12) est réalisée en forme de cellule à cristaux liquides en matière plastique.

6. Dispositif de protection solaire selon l'une des revendications précédentes,
**caractérisé par**
une monture de lunettes (22) prévue pour loger le filtre de protection solaire optique (12).

7. Dispositif de protection solaire selon l'une des revendications précédentes,
**caractérisé en ce que**
l'au moins une unité captrice (16) est prévue, en au moins un état de fonctionnement, pour au moins partiellement fournir une énergie pour l'actionnement de l'au moins une cellule à cristaux liquides (14, 14') du filtre de protection solaire optique (12).

8. Dispositif de protection solaire selon l'une des revendications précédentes,
**caractérisé en ce que**
l'au moins une unité captrice (16) comprend au moins une photodiode (24).

9. Procédé de fonctionnement d'un dispositif de protection solaire (10) selon l'une des revendications précédentes.

10. Procédé selon la revendication 9,
**caractérisé en ce que**
l'au moins une cellule à cristaux liquides (14, 14') du filtre de protection solaire optique (12) est actionnée et/ou régulée pour générer un gradient de perméabilité (20) définie pour un utilisateur (31).

11. Procédé selon la revendication 9 ou 10,
**caractérisé en ce que**
l'au moins une cellule à cristaux liquides (14, 14') du filtre de protection solaire optique (12) est actionnée et/ou régulée pour générer, en fonction d'un rayonnement solaire, un gradient de perméabilité (20) définie pour un utilisateur (31).

12. Procédé selon l'une des revendications 9 à 11,
**caractérisé en ce que**
l'au moins une cellule à cristaux liquides (14, 14') du filtre de protection solaire optique (12) est actionnée et/ou régulée pour générer, en fonction d'au moins une entrée d'utilisateur, un gradient de perméabilité (20) définie pour un utilisateur (31).

13. Procédé selon la revendication 12,
**caractérisé en ce que**
pour adapter le gradient de perméabilité (20), l'au moins une cellule à cristaux liquides (14, 14') du filtre de protection solaire optique (12) est actionnée et/ou régulée pour générer, en fonction d'un étage réglable par l'utilisateur, un gradient de perméabilité (20) définie pour un utilisateur (31), ledit étage étant sélectionnable moyennant une unité d'entrée.
